# EUROPEAN PATENT APPLICATION

(11) **EP 1 188 838 A1**
(43) Date of publication of application: **20.03.2002**
(21) Application number: 00936911.7
(22) Date of filing: 13.06.2000
(51) Int. Cl.: C12P 35/00

(54) **METHOD FOR PRODUCING 7-AMINODESACETOXYCEPHALOSPORANIC ACID (7-ADCA)**

(30) Priority: 18.06.1999 ES 9901365
(71) Applicant: Antibioticos, S.A.U., 24080 Leon (ES)
(72) Inventor: VELASCO ALVAREZ, Javier, E-24080 Leon (ES); ADRIO FONDEVILA, José, E-24080 Leon (ES); MORENO VALLE, Miguel Angel, E-24080 Leon (ES); DIEZ GARCIA, Bruno, E-24080 Leon (ES); ESTEBAN MORALES, Manuel, E-24080 Leon (ES); BERNASCONI, Ermanno, E-24080 Leon (ES); BARREDO FUENTE, José Luis, E-24080 Leon (ES)
(74) Representative: Elzaburu, Alberto de
(86) International application number: ES0000211
(87) International publication number: WO0078989

(57) **Abstract**

A method of obtaining 7-aminodesacetoxycephalosporanic acid (7-ADCA). The method is based on the use of desacetoxycephalosporin C (DAOC) produced directly by fermentation of Acremonium chrysogenum (also called Cephalosporium acremonium) and catalysed by the enzymatic activities D-amino-acid oxidase (DAO) and glutaryl-7-ACA acylase (GLA). The method of production of DAOC by fermentation of A. chrysogenum is based on inactivation of the cefEF gene and subsequent expression of the cefE gene giving rise to the strain A. chrysogenum △cefEF-cefE. The invention includes the following sections: (I) inactivation of the cefEF gene of A. chrysogenum, which codes for the enzymatic activities desacetoxycephalosporin C synthase (DAOCS or expandase) and desacetylcephalosporin C synthase (DACS or hydroxylase), (II) expression of the cefE gene, which codes for the enzymatic activity DAOCS, derived from an actinomycete in the transformant of A. chrysogenum inactivated in the cefEF gene, (III) development of a method of fermentation of the recombinant strain for the production of DAOC and (IV) enzymatic conversion of DAOC to 7-ADCA catalysed by the enzymes DAO and GLA. Alternatively, it would be possible to produce 7-ADCA directly by fermentation of A. chrysogenum by combined expression of the genes that code for DAO and GLA in the DAOC-producing strain A. chrysogenum △cefEF-cefE or by expression of a gene that codes for the enzyme cephalosporin acylase in A. chrysogenum △cefEF-cefE.

## Description

### FIELD OF THE INVENTION

The invention relates to the production of 7-ADCA, or its intermediates penicillin N or desacetoxycephalosporin C, by fermentation of transformed Acremonium chrysogenum strains.

### STATE OF THE ART

The cephalosporins are a group of antibiotics of considerable therapeutic importance that are produced industrially by fermentation of A. chrysogenum or alternatively by chemical expansion of the thiazolidine ring of the penicillins, generating a dihydrothiazine ring. By incorporating side chains at various positions of the β-lactam and dihydrothiazine rings, it is possible to synthesize an enormous number of cephalosporins with greater antibiotic capacity or improved pharmacokinetic properties. Preparation of these semisynthetic antibiotics has aroused considerable interest in recent years, leading to optimization and innovation in the processes and methods used for their preparation.

7-Aminodesacetoxycephalosporanic acid (7-ADCA) is one of the substrates used for the preparation of cephalosporins characterized by the absence of functionalization at the 3 position. Owing to this absence of functionalization, generally 7-ADCA is synthesized by expansion of the thiazolidine ring of penicillins obtained by fermentation, whereas the other cephalosporanic compounds are prepared starting from natural cephalosporins obtained by fermentation. Usually 7-ADCA is produced starting from penicillin G or V, requiring from 4 to 5 chemical steps for expanding the 5-membered ring characteristic of the penicillins to the 6-membered ring characteristic of the cephalosporins. The said process, like many chemical processes, has a number of disadvantages in comparison with biological processes: it is a complex process with multiple stages, it requires the use of chemical reagents that are both toxic and expensive, it uses organic solvents that are harmful to the environment, and during the process there is formation of by-products and impurities that require complex purification systems. As an alternative to the chemical process, various strategies have been described for production of 7-ADCA based on the fermentation of microorganisms and bioconversion. This relates to methods equivalent to those developed in the 1980s for the production of 6-aminopenicillanic acid (6-APA) by enzymatic hydrolysis of penicillins G and V or the strategies described in the 1990s for the production of 7-aminocephalosporanic acid (7-ACA) by enzymatic hydrolysis of the side chain of D-(α)-aminoadipic acid of cephalosporin C. In this last case the reaction is catalysed by the enzymes D-amino-acid oxidase (DAO), which participates in the transamination that transforms the side chain of aminoadipic acid to glutaric acid, and glutaryl-7-ACA acylase, which hydrolyses the side chain of glutaric acid generating 7-ACA.

Production of strains of A. chrysogenum that are overproducers of desacetoxycephalosporin C (DAOC), the direct precursor of 7-ADCA, is complex owing to the existence in the said microorganism of the bifunctional enzyme desacetoxycephalosporin C synthase (DAOCS or expandase), which expands and hydroxylates the thiazolidine ring generating desacetylcephalosporin C (DAC). An equivalent problem arises in the case of actinomycetes, where the presence of enzymes capable of methoxylating the 7 position of the cephalosporin molecule forming cephamycins, hampers the production of strains that are overproducers of DAOC. Most attempts to obtain 7-ADCA by means of an enzymatic or fermentation process have focused on the use of enzymes with DAOCS activity, which catalyse the expansion of the 5-membered thiazolidine ring present in the penicillins to the 6-membered dihydrothiazine ring present in the cephalosporins.

Theoretically, the 6-APA produced directly by fermentation and/or enzymatic hydrolysis of penicillins G or V could be expanded to 7-ACA by the use of DAOCS, but unfortunately none of the DAOCS described up to now recognize 6-APA as a substrate and are therefore unable to effect the expansion of the thiazolidine ring. On the other hand, the possibility of expanding penicillins G or V, both in vitro and in vivo, by means of a DAOCS to form the corresponding cephalosporins G or V, which can be hydrolysed enzymatically to 7-ADCA, has been described. Nevertheless, owing to the high specificity of DAOCS substrate, the efficiencies and productivities of these systems are a very long way from industrial application.

The production of adipoyl-7-ADCA by using a recombinant strain of P. chrysogenum, in which DAOCS of S. clavuligerus had been expressed, has recently been described. Addition of adipic acid during fermentation (instead of the phenylacetic or phenoxyacetic acids that are used in the production of penicillins G or V respectively) gives rise to the production of adipoyl-6-APA, a molecule that is recognized in vivo by DAOCS, causing expansion of the thiazolidine ring and producing adipoil-7-ADCA. Then the adipoyl-7-ADCA is purified of the other components of the fermentation medium (in particular adipoyl-6-APA) and is hydrolysed enzymatically using an adipyl acylase, generating 7-ADCA. Although the said process has high efficiencies both of production and of enzymatic expansion and hydrolysis of the side chain, it has the following principal drawbacks: (I) the need to add a high-priced side chain such as adipic acid and (II) the production of mixtures of adipoyl-6-APA and adipoyl-7-ADCA which, owing both to the similarity of their structure and to their behaviour in enzymatic hydrolysis with adipyl acylase, have to be separated beforehand, which brings an added complexity to the process.

The biosynthetic pathways of penicillins, cephalosporins and cephamycins (diagram 1) have been the subject of detailed investigations in recent years, so that a thorough understanding of the biosynthetic steps involved has been achieved. The β-lactam antibiotics are derived from three precursor amino acids: L-α-aminoadipic acid, L-cysteine and L-valine, which are condensed to form the precursor tripeptide δ-(L-α-aminoadipyl)-L-cysteinyl-D-valine (LLD-ACV) by the enzyme ACV synthetase (ACVS). Next, the linear tripeptide is cyclized oxidatively by the action of the enzyme isopenicillin N synthase (IPNS) giving rise to isopenicillin N (IPN). This compound possesses weak antibiotic activity and is the precursor both of penicillins and of cephalosporins and cephamycins. Transformation of IPN to penicillin G takes place by exchange of the side chain of α-aminoadipic acid with another of phenylacetic acid, previously activated as phenylacetyl-CoA, in a reaction catalysed by the enzyme acyl-CoA:6-APA acyltransferase.

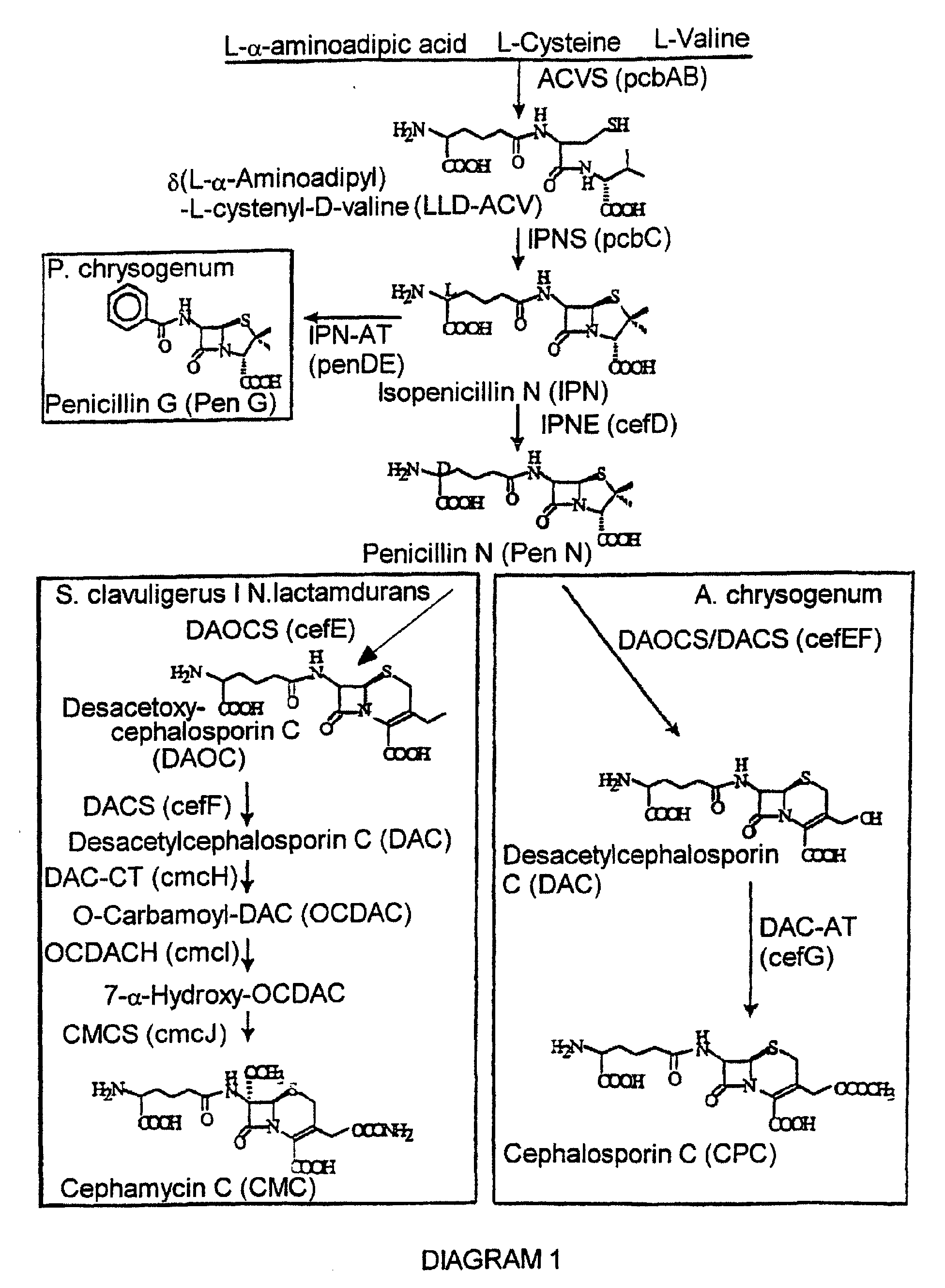

For their part, the biosynthesis of cephalosporins and cephamycins starting from IPN begins with isomerization of the side chain of L-α-aminoadipyl to D-α-aminoadipyl, catalysed by the enzyme IPN epimerase (IPNE). The penicillin N formed is converted to DAOC by the action of the enzyme DAOCS, which converts the 5-membered thiazolidine ring typical of the penicillins to the 6-membered dihydrothiazine ring of cephalosporins and cephamycins. The next reaction consists of hydroxylation of the DAOC molecule to generate desacetylcephalosporin C (DAC) and is catalysed by the enzyme DAC synthetase (DACS) or hydroxylase. Acetylation of DAC with consequent formation of cephalosporin C (CPC) is the final biosynthetic step in the cephalosporin-producing fungi. This reaction is catalysed by the enzyme DAC acetyltransferase.

Most of the genes that code for enzymes involved in the biosynthesis of cephalosporin in A. chrysogenum have been identified: pcbAB codes for ACVS (Gutierrez et al. 1991. J. Bacteriol. 173: 2354-2365), pcbC codes for IPNS (Samson et al. 1985. Nature 318: 191-194), cefEF codes for DAOCS/DACS (Samson et al. 1987. Biotechnology 5: 1207-1214) and cefG codes for DAC acetyltransferase (Gutiérrez et al. 1992. J. Bacteriol. 174: 3056-3064). The genes that code for the first two enzymes of the biosynthetic pathway (pcbAB and pcbC) are grouped in chromosome VI, whereas cefEF and cefG, which code for the enzymes that catalyse the last two steps of the pathway, are grouped in chromosome II (Skatrud and Queener, 1989. Gene 78: 331-338). However, the gene of A. chrysogenum that codes for the enzymatic activity IPNE equivalent to the gene cefD described in actinomycetes (Coque et al. 1993. Mol. Gen. Genet. 236: 453-458; Kovacevic et al. 1990. J. Bacteriol. 172: 3952-3958) has not yet been identified. The DAOCS and DACS activities are localized in a single polypeptide (Scheidegger et al. 1984. J. Antibiot. 37: 522-531) coded by the cefEF gene in A. chrysogenum (Samson 1987. Biotechnology 5: 1207-1214), whereas in cephamycin-producing microorganisms the said activities are localized in two separate polypeptides coded respectively by the genes cefE (Kovacevic et al. 1989. J. Bacteriol. 171: 754-760; Barredo et al. 1991. Microbiología 7: 1-12; Ingolia T.D. et al. 1991. US5070020; Coque et al. 1993. Mol. Gen. Genet. 236: 453-458; Kimura et al. 1996. Appl. Microbiol. Biotechnol. 44: 589-596; Enguita et al. 1998. J. Bacteriol. 180: 5489-5494) and cefF (Kovacevic et al. 1991. J. Bacteriol. 173: 398-400). Industrial programmes for improvement of strains of A. chrysogenum have given rise to chromosomal reorganizations, which produce altered electrophoretic patterns in strains with different production of cephalosporin, without detailed knowledge of the relation that exists between the said translocations and the level of production of antibiotic (Mathison et al. 1993. Curr. Genet. 23: 33-41).

The biosynthetic genes of cephalosporin have been used previously for improving the production of an industrial strain of A. chrysogenum (Skatrud et al. 1989. Biotechnology 7: 477-485). In the said strain, which accumulated a considerable quantity of penicillin N, additional copies of the cefEF and cefG genes were introduced by transformation, resulting in increasing DAOCS enzymatic activity, reducing the quantity of penicillin N accumulated and greatly increasing the production of CPC. Improvements in production of CPC in A. chrysogenum by increasing the available oxygen as a result of the expression of a bacterial haemoglobin derived from Vitreoscilla have also been described (DeModena et al. 1993. Biotechnology 11: 926-929). Furthermore, strains of A. chrysogenum capable of producing penicillin G through heterologous expression of the penDE gene of Penicillium chrysogenum have been developed (Gutiérrez et al. 1990. Mol. Gen. Genet. 225: 56-64). The penDE gene codes for the enzymatic activity acyl-CoA:6-APA acyltransferase, which is able to exchange the side chain of α-aminoadipic acid present in the molecule of isopenicillin N with another consisting of aromatic acids, for example phenylacetic acid. Another type of transformants of A. chrysogenum of considerable interest are those that are able to produce 7-aminocephalosporanic acid (7-ACA) directly (Isogai et al. 1991. Biotechnology 9: 188-191). In this case, A. chrysogenum was transformed with the genes that code for the activities D-amino-acid oxidase (DAO) of Fusarium solani and glutaryl-7-ACA acylase (GLA) of Pseudomonas diminuta. The said transformants produce small quantities of 7-ACA, 7-ADCA and 7-aminodesacetylcephalosporanic acid.

One of the compounds of greater interest in the pharmaceutical industry is 7-ADCA, which is used as substrate for the production of semisynthetic cephalosporins. The most widely used industrial process for the production of 7-ADCA consists of expansion of the thiazolidine ring of penicillin by chemical methods. The starting point for the chemical synthesis of 7-ADCA is an aqueous solution of penicillin, which is oxidized to penicillin sulphoxide with a peroxide and then its ring of 5 carbon atoms expands forming a ring of 6 atoms (7-phenylacetamido desacetoxycephalosporanic acid or FADCH), in the presence of a suitable chemical catalyst. Purified FADCH is submitted to an enzymatic treatment catalysed by the enzyme penicillin amidase or penicillin acylase for removing the amide bond present in its molecule. 7-ADCA and the side chain are obtained as reaction products. The last step consists of purification of 7-ADCA.

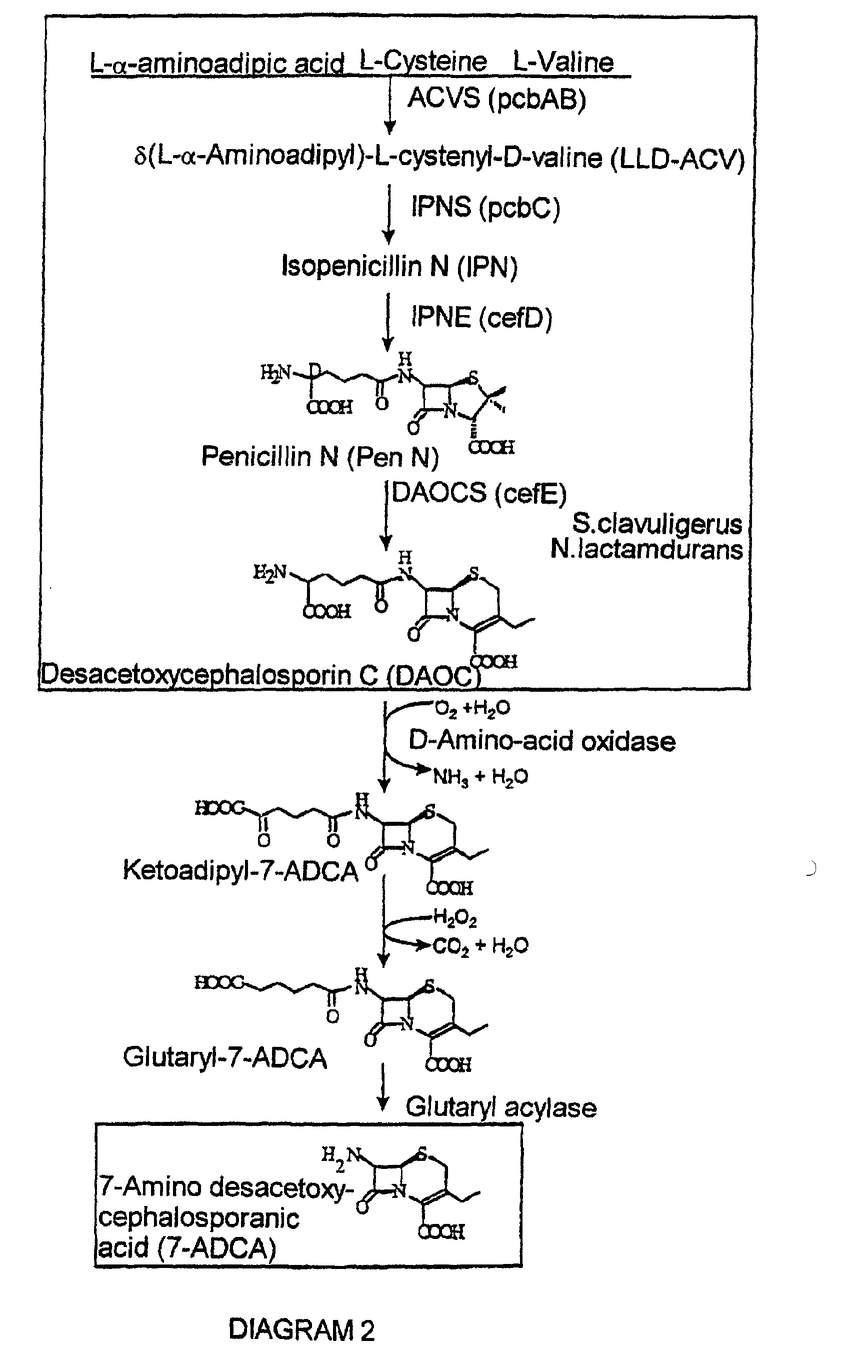

One of the keys to replacement of the chemical method described above with a biological process of fermentation or bioconversion consists of characterization of the genes and enzymes involved in the biosynthesis of cephalosporins and cephamycins. In this direction, purification of the enzymatic activities IPNS, IPNE and DAOCS starting from acellular extracts of various prokaryotic organisms including S. clavuligerus has been described (Wolfe et al. 1985. US4510246; Wolfe et al. 1985. US4536476). In addition, the cloning and characterization of the cefE genes, which code for DAOCS, of S. clavuligerus and Nocardia lactamdurans has been described (Kovacevic et al. 1989. J. Bacteriol. 171: 754-760; Coque et al. 1993. Mol. Gen. Genet. 236: 453-458).

A. chrysogenum expresses the DAOCS enzyme that is able to expand the thiazolidine ring of penicillins to the dihydrothiazine ring of cephalosporins. The cefEF gene, which codes for the bifunctional enzyme DAOCS/DACS, is present in this microorganism. Therefore the final product of this enzyme is DAC and not DAOC as occurs in the cephamycin-producing organisms, which have separate DAOCS and DACS enzymes. Various types of penicillins have been treated with an acellular extract of A. chrysogenum, obtaining the corresponding cephalosporins (Demain et al. 1979. US4178210; Demain et al. 1979. US4248966; Demain et al. 1981. US4307192). Furthermore, a number of parameters of the DAOCS/DACS enzyme of A. chrysogenum have been determined, such as isoelectric point, molecular weight, amino acid composition, ratio of hydroxylase/expandase activities and peptide fragmentation (Wu-Kuang et al. 1988. US 4753881). Although considerable effort has been expended for obtaining enzymes that possess separate expandase and hydroxylase activities, and which could therefore expand penicillin N to DAOC, the results obtained so far have been completely without success. The possibility of separating the genetic information coding for DAOCS and DACS and expressing the two activities separately has been suggested, though no description exists in this respect.

Various attempts have been described for achieving enzymatic expansion of penicillin G or V to their corresponding desacetoxycephalosporin G or V using DAOCS from various sources. Optimization of the expansion in vitro of a large number of penicillins, including penicillin G, by using a free extract of cells of S. clavuligerus NP1, a mutant that is deficient in production of cephalosporins, has recently been described (Cho et al. 1998. Proc. Natl. Acad. Sci. USA. 11544-11548). Although in this case there is a considerable increase in yields of expansion through adjustment of the reaction conditions, the said results are a long way from possible industrial use owing to the nature and cost of the co-factors, low yields of the expansion process and difficulties in isolating and purifying cephalosporin G in the presence of appreciable concentrations of unexpanded penicillin G.

The natural DAOCS described up to now act efficiently on penicillin N, an intermediate in the biosynthesis of cephalosporins and cephamycins that is not available commercially, but not on the penicillins produced industrially by P. chrysogenum: penicillin G and penicillin V. The idea of modifying the substrate specificity of the DAOCS enzyme with the aim of being able to use penicillin G as substrate and expand it to phenylacetyl-7-ADCA has been shared for years by a number of research teams (Cantwell et al. 1990. Curr. Genet. 17: 213-221), nevertheless its achievement has not yet been described. The aromatic side chain of phenylacetyl-7-ADCA could easily be eliminated by means of the enzyme penicillin acylase, producing 7-ADCA. In addition, the cefD and cefE genes of S. clavuligerus, which code for the enzymatic activities IPNE and DAOCS, have been expressed simultaneously in P. chrysogenum producing small amounts of DAOC owing to isomerization of IPN to penicillin N and subsequent formation of the 6-membered ring characteristic of the cephalosporins (Cantwell et al. 1992. P. Roy. Soc. Lond. B 248: 283-289). The cloning and characterization and expression in E. coli of the cefE gene of clavuligerus, which codes for DAOCS activity, has been described previously (Kovacevic et al. 1989. J. Bacteriol. 171: 754-760). In addition, the DNA sequence of the cefE gene of S. clavuligerus is known, and it is known that its expression in P. chrysogenum generates DAOCS activity in the said fungus (Ingolia T.D. et al. 1991. US5070020; Queener et al. 1994. Ann. N. Y. Acad. Sci. 721: 178-193). For its part, the cefEF gene of A. chrysogenum has been expressed in P. chrysogenum, although, just as in the case of the cefE gene of S. clavuligerus, up to now the conversion of penicillin G or V to their corresponding cephalosporins using these recombinant strains of P. chrysogenum has not been demonstrated.

Another strategy employed, in view of the difficulty of direct expansion of penicillins G or V to the corresponding cephalosporins, has been the expansion of penicillins with different side chains to the phenylacetic or phenoxyacetic acids. Addition of phenylacetic or phenoxyacetic acids to fermentations of P. chrysogenum gives rise to the biosynthesis of penicillin G or penicillin V respectively. Moreover, when adipic acid is added to the culture medium, P. chrysogenum produces adipyl-6-APA or carboxy-n-butyl-penicillin (Ballio et al. 1960. Nature 185: 97-99). The adipyl-6-APA produced by P. chrysogenum is an efficient substrate for DAOCS since its side chain (adipic acid) is equivalent to that of penicillin N, the natural substrate of DAOCS. Taking these studies as a basis, a process that can be used industrially for the biosynthesis of 7-ACA and 7-ADCA was described for the first time (Crawford et al. 1995. Biotechnology 13: 58-62; Bovenberg et al. 1998. WO9802551 A2). In this case transformants of industrial strains of P. chrysogenum that expressed the cefE gene of S. clavuligerus were selected, which were able to produce adipyl-7-ADCA in a proportion of 17% relative to the production of penicillin V of the parent strain. As was to be expected, the side chain of adipic acid was eliminated efficiently by treatment with the enzymatic activity cephalosporin acylase of Pseudomonas. Similarly, transformants of P. chrysogenum that expressed the cefEF gene (DAOCS-DACS) of C. acremonium produced both adipyl-7-ADCA and adipyl-7-aminodesacetylcephalosporanic acid (adipyl-7-ADACA). In addition, transformants of P. chrysogenum that expressed the cefEF and cefG genes (DAC acetyltransferase) produced a mixture of adipyl-7-ADCA, adipyl-7-ADACA and adipyl-7-ACA. Although they obtained relatively small amounts of these adipyl derivatives (0.6% of penicillin V produced in fermentations with addition of phenoxyacetic acid), the authors suggest that by means of classical techniques for improvement of strains it could be possible to obtain productivities that would permit its application on an industrial scale (Crawford et al. 1995. Biotechnology 13: 58-62).

The method adopted in the present patent consists of obtaining a strain of A. acremonium whose biosynthetic pathway of CPC is blocked at the level of DAOCS/DACS enzymatic activity, so that it produces penicillin N. This strain is obtained by inactivation of the function of the cefEF gene by gene disruption by a process of double recombination with a plasmid in which the cefEF gene is interrupted. Inactivation of the function of the gene by disruption is a technique used for the modification of biosynthetic pathways and the accumulation of biosynthetic intermediates that can be used for obtaining certain secondary metabolites. However, there are few cases in which this technique has been employed successfully in industrial strains of filamentous fungi. As an example, in P. chrysogenum we can cite disruption of the lys2 gene, obtaining strains that are lysine auxotrophs and are able to biosynthesize a larger amount of α-aminoadipic acid, which is a precursor in the biosynthesis of penicillin (Casqueiro et al. 1997. Spanish patent P9701343. Casqueiro et al., 1999. J. Bacteriol. 181: 1181-1188). In the case of A. nidulans, the phacA gene has been disrupted, achieving a decrease in phenylacetate hydroxylase activity, therefore limiting the oxidation of phenylacetic acid, and promoting its incorporation in penicillin G (Fernández-Cañón et al., 1997. Spanish Patent P9700833; Fernández-Cañón et al., 1998. PCT/ES98/0010; Mingot et al., 1999. J. Biol. Chem. 274: 14545-14550). In A. chrysogenum, inactivation of the genes pcbAB (Hoskins et al. 1990. Curr. Genet. 18: 523-530), pcbC (Waltz and Kück. 1993. Curr. Genet. 24: 421-427) and cefG (Matsuda et al. 1992. Biochem. Biophys. Res. Commun. 186: 40-6) has been described, in all cases obtaining transformants incapable of producing cephalosporin C. Despite the fact that A. chrysogenum is a species in which gene disruptions cannot be obtained easily (Waltz and Kück. 1993, Curr. Genet. 24: 421-427), inactivation by disruption of the cefEF gene is described for the first time in the present patent.

The strain of A. acremonium with the function of the cefEF gene inactivated is then transformed with a plasmid that includes the cefE gene derived from an actinomycete expressed under the control of various fungal promoters. Finally the transformants that produce a larger amount of DAOC are selected, and this is converted to 7-ADCA by a two-stage enzymatic process: (I) Conversion of DAOC to 7-β-(5-carboxy-5-oxopentanamido)-desacetoxycephalosporin(ketoadipyl-7-ADCA) catalysed by DAO activity. The ketoadipyl-7-ADCA formed reacts non-enzymatically with hydrogen peroxide to give rise to 7-β-(carboxybutanamido)-desacetoxycephalosporin (glutaryl-7-ADCA or GL-7-ADCA). (II) The glutaryl residue of GL-7-ADCA is eliminated by treatment with the GLA enzyme generating 7-ADCA. Overall, the process described makes it possible to obtain 7-ADCA by a completely biological route and without the use of organic solvents.

Up to now there has been no description of disruption of the cefEF gene of A. chrysogenum with the aim of increasing the production of penicillin N, in view of the scant commercial value of this antibiotic owing fundamentally to: (I) the scant antimicrobial activity displayed by penicillin N compared with the hydrophobic penicillins (G or V), semisynthetic penicillins and cephalosporins, (II) its scant usefulness as intermediate in the preparation of 6-APA because of its reduced stability, as well as its physicochemical properties of solubility and acidity-basicity which complicate its isolation enormously and (III) the impossibility of eliminating its side chain with the conventional acylases of penicillin G or V. Only the existence of mutants of A. chrysogenum capable of accumulating penicillin N is known, and these have been used for characterization of the biosynthetic pathway of cephalosporin. On the other hand, the accumulation of DAOC together with DAC and CPC in fermentations of A. chrysogenum has been described. In this case the DAOC is regarded as an undesirable contaminant which complicates the industrial production of CPC with an acceptable degree of purity.

The use of DAOC as an intermediate in the production of 7-ADCA has not been described up to now owing to the impossibility of obtaining DAOC as a single product from the fermentation of A. chrysogenum. In all cases a mixture of DAOC and DAC is produced, and these are then difficult to separate. In addition, the difficulty of eliminating, until a short time ago, the side chain of aminoadipic acid from the DAOC molecule using enzymatic methods, meant that DAOC was a molecule that was not suitable for the industrial manufacture of 7-ADCA. However, construction of the A. chrysogenum strain △cefEF-cefE makes increased production of DAOC possible, the latter being the principal cephalosporin. The DAOC produced can be converted to 7-ADCA by using the enzymes DAO and GLA by methods equivalent to those described previously (Croux et al. 1994. US5354667; Cambiaghi et al. 1991. EP0496993; García et al. 1998. EP0843015).

### DETAILED DESCRIPTION OF THE INVENTION

### EXAMPLE 1. Disruption of the cefEF gene in A. chrysogenum.

### 1.1. Construction of pALC73, plasmid for inactivating the cefEF gene of A. chrysogenum.

With the aim of inactivating the cefEF gene of A. chrysogenum and obtaining a mutant △cefEF that will accumulate penicillin N, the technique of gene disruption by double recombination called "one step gene disruption" was used (Rothstein, 1983. Meth. Enzymol. 101: 202-211). The result of this technique is to obtain a stable disrupted transformant, as directly repeated copies are not generated in the recombination zone. The method is based on incorporating a marker for selecting transformants in a restriction site of the gene of interest, so that the reading frame of the said gene is disrupted. The marker gene must possess, on both sides, a DNA fragment homologous to the target sequence of a sufficient size to permit crossing over. A minimum size of 500 pb has been described for these homologous sequences (Rothstein, 1991. Meth. Enzymol. 194: 281-301).

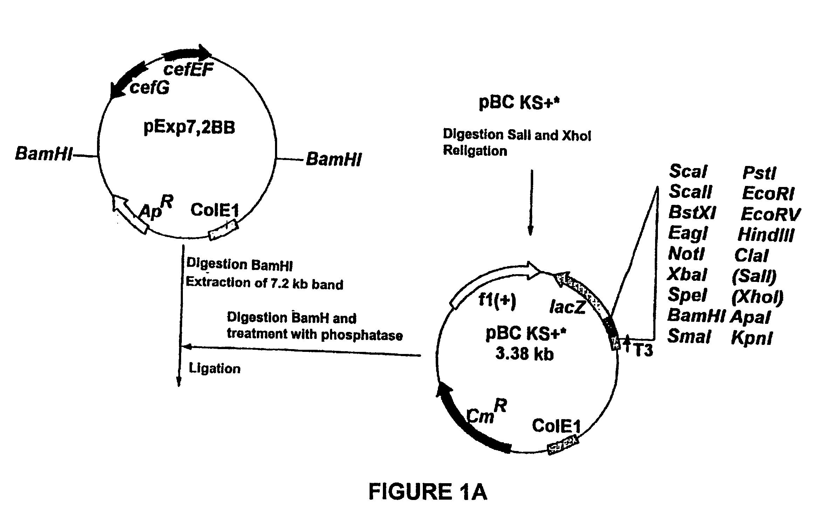

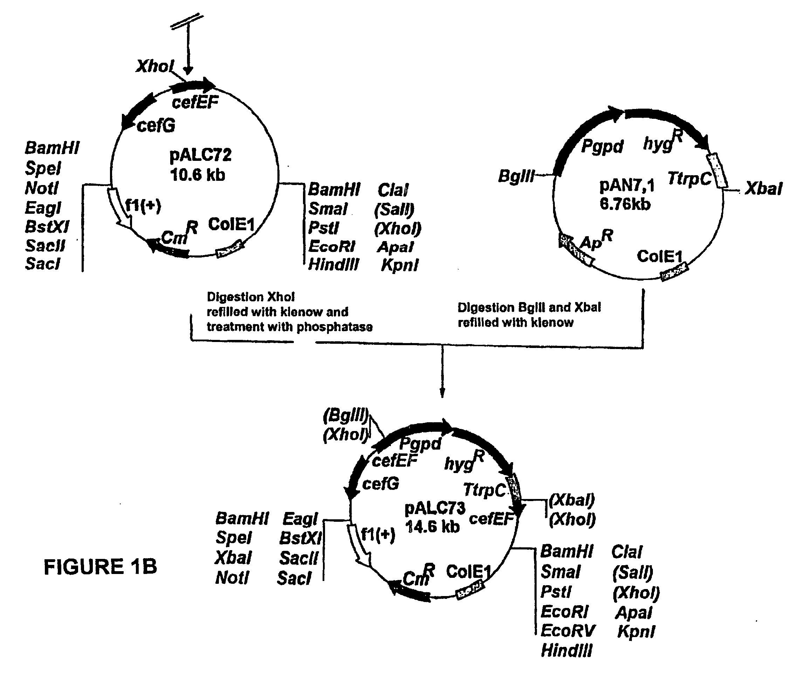

In the present example, the hygromycin resistance cassette, formed by the promoter of the gpd gene (glyceraldehyde-3-phosphate dehydrogenase) of A. nidulans, the hygromycin resistance gene (hyg^{r}) and the terminator of the trpC gene (tryptophan C), were integrated in the XhoI site of the cefEF gene (Fig. 1A-B). To do this, in the first place the XhoI site present in pBC KS (+) was eliminated by double digestion SalI-XhoI and religation, giving rise to the plasmid pBC* KS (+) which lacks the restriction sites XhoI and SalI. Then a BamHI fragment of 7.2 kb which includes the cefEF and cefG genes of A. chrysogenum was inserted at pBC* KS (+), giving rise to the plasmid pALC72 (Fig. 1A-B). Finally, the BglII-XbaI fragment of the pAN7.1 plasmid, which includes the hygromycin resistance cassette and whose ends had previously been blunted with Klenow, was inserted in the XhoI site inside the cefEF gene of pALC72, obtaining the plasmid pALC73 (Fig. 1A-B). In this way, the selection marker is flanked by two DNA fragments of 2.2 kb and 5.0 kb homologous to the region of the genome of A. chrysogenum that contains the cefEF and cefG genes respectively. The transformants of A. chrysogenum with pALC73 were obtained in accordance with methods previously described (Queener et al. 1985. Lieve (ed.) Microbiology-1985. pp. 468-472. ASM. Washington) and were selected for resistance to 5-10 µg/ml of hygromycin. The strain that includes the plasmid pALC73, designated E. coli TOP10/pALC73 was deposited in the Spanish collection of Standard Cultures (Colección Española de Cultivos Tipo, CECT), Valencia University, Research Building, Burjasot Campus, 46100 Burjasot (Valencia) with No. 5151.

### 1.2. Preselection of transformants for loss of production of cephalosporin.

The transformants selected for hygromycin resistance were grown on plates of solid medium LPE (Le Page & Campbell, 1946, J. Biol. Chem. 162: 163-171), incubating for 7 days at 28°C. Then two cylinders or plugs of each transformant were removed using a punch. With the aim of determining the production of cephalosporin, one of the plugs was bioassayed on a plate of LB medium to which were added a culture of E. coli sensitive to β-lactams (E. coli ESS) and a preparation of penicillinase type I (Sigma P-0389) to a final concentration of 0.1 mg/ml. The other plug was assayed in a plate of LB medium with E. coli ESS but without adding penicillinase, with the aim of determining the presence both of cephalosporin and of penicillin. Those transformants that produced an inhibition halo on the plates without penicillinase and did not produce a halo on the plates with penicillinase, indicating that they were unable to synthesize cephalosporins, were selected.

The results obtained are shown in the following table:

| Transformant No. | Hygromycin resistance (µg/ml) | Halo without penicillinase (mm) | Halo with penicillinase (mm) |
|---|---|---|---|
| 2672* | 5 | 12 | 0 |
| 2673* | 10 | 16 | 0 |
| 2674 | 10 | 11 | 12 |
| 2675* | 10 | 11 | 0 |
| 2676 | 5 | 15 | 12 |
| 2677 | 5 | 13 | 11 |
| 2678 | 5 | 14 | 11 |
| 2679 | 5 | 17 | 11 |
| 2680* | 10 | 14 | 0 |
| 2681 | 5 | 17 | 12 |
| 2682 | 5 | 14 | 12 |
| 2683 | 10 | 14 | 12 |
| 2684 | 5 | 15 | 12 |
| 2685 | 5 | 16 | 12 |
| 2686 | 5 | 15 | 12 |
| 2687 | 5 | 18 | 12 |
| 2688 | 5 | 17 | 12 |
| 2689 | 5 | 16 | 12 |
| 2690 | 5 | 15 | 12 |
| 2691* | 10 | 15 | 0 |
| 2692* | 10 | 14 | 0 |
| 2693* | 5 | 18 | 0 |
| 2694* | 10 | 17 | 0 |
| 2695* | 10 | 15 | 0 |
| 2696* | 10 | 14 | 0 |
| 2697* | 10 | 14 | 0 |
| 2698* | 10 | 13 | 0 |
| 2699* | 10 | 12 | 0 |
| 2700 | 10 | 12 | 12 |

The transformants 2672, 2673, 2675, 2680, 2691, 2692, 2693, 2694, 2695, 2696, 2697, 2698 and 2699 (marked with an asterisk) possessed the required characteristics and were selected for subsequent tests.

### 1.3. Test for inactivation of the cefEF gene by the Southern test.

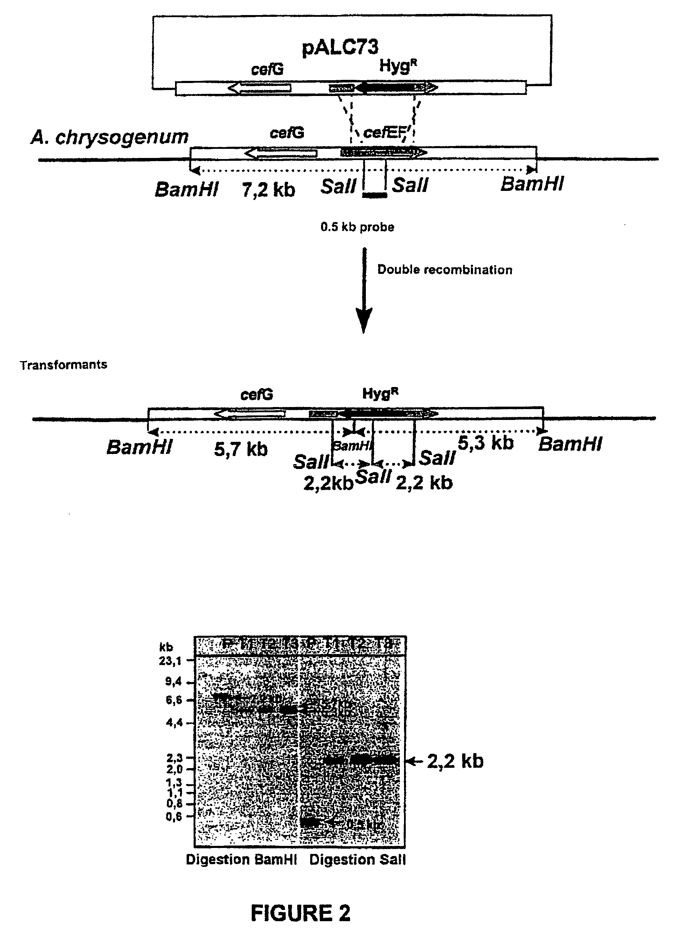

The cefEF gene of A. chrysogenum is located in a BamHI fragment of genomic DNA of 7.2 kb. Homologous double recombination between the pALC73 plasmid and the fragments adjacent to the cefEF gene ought to have caused modification of the restriction template previously mentioned, it being possible to visualize the said change by means of a Southern analysis (Fig. 2). For this, total DNA was purified from the A. chrysogenum parent strain and from 3 of the transformants selected by bioassay (2691, 2696 and 2698) as is indicated in the previous section. The total DNA was digested with the enzymes BamHI and Sail and, after electrophoretic separation and transfer to nitrocellulose filters, was hybridized using as probe a 0.5 kb DNA fragment SalI within the cefEF gene. The result obtained (Fig. 2) shows the expected change of restriction template: with the A. chrysogenum parent strain, a hybridization band of 7.2 kb BamHI was obtained, whereas two bands of 5.7 kb and 5.3 kb respectively appeared in the transformants. In addition, the results with the SalI enzyme confirmed the inactivation of the cefEF gene: a hybridization band of 0.5 kb in the A. chrysogenum parent strain and a double band of 2.2 kb in the disrupted transformants. The strains disrupted in the cefEF gene were designated A. chrysogenum △cefEF T1, A. chrysogenum △cefEF T2 and A. chrysogenum △cefEF T3.

### EXAMPLE 2. Production of penicillin N by A. chrysogenum △cefEF.

### 2.1. Fermentation of A. chrysogenum and A. chrysogenum △cefEF.

For fermentation of the A. chrysogenum parent strain and of the strains carrying the inactivated cefEF gene A. chrysogenum △cefEFT1, A. chrysogenum △cefEFT2 and A. chrysogenum △cefEFT3, slants of solid medium LPE were sown (Le Page & Campbell, 1946, J. Biol. Chem. 162: 163-171) and were incubated for 7 days at 28°C until a sporulated culture was obtained. Fermentation in liquid medium was started by inoculating the spores obtained from a slant in a 250-ml flask that contained 50 ml of complex inoculation medium (Shen et al., 1986, Bio/Technology 4: 61-64). This flask was incubated for 48 hours at 28°C and 250 rpm to obtain an abundant vegetative mycelium. Fermentation in conditions of production of antibiotics was started on inoculating 0.5 ml (3%) of the previous vegetative culture in a 500-ml flask with 15 ml of fermentation complex medium (Shen et al., 1986, Bio/Technology 4: 61-64). This culture was incubated for 7 days at 25°C and 250 rpm, collecting 1 ml samples every 24 hours for investigating the production of antibiotics in the course of fermentation. Next, analyses were undertaken with the aim of determining: (I) that the antibiotic produced by the transformants A. chrysogenum △cefEF was penicillin N, (II) the level of production of penicillin N of these transformants and (III) possible presence of other antibiotics in the fermentation medium. The analyses undertaken, as described below, were the following: HPLC, enzymatic expansion in vitro and LC masses.

### 2.2. Determination of production of antibiotics by HPLC.

The fermentation samples were centrifuged for 5 minutes at 20 000 g to remove the mycelium and then 50 µl of the supernatant was diluted 10 times with water and analysed in a Waters TAD chromatograph (Waters Associates, Milford, MA) with 486M1 detector, W600 pump, 717 autoinjector and a Nucleosil C₁₈-10 µm column (250x4.6 mm) (Phenomenex, Torrance, CA). The samples were analysed at a flow rate of 2.3 ml/min and variable wavelength (214 for detecting penicillins or 254 nm for detecting cephalosporins). Elution was effected using, as the mobile phase, ammonium formate pH 3.3-methanol-tetrahydrofuran (99.85: 0.1: 0.05) in isocratic mode for 30 minutes. Assignment of the peaks obtained to each compound was carried out by comparison with reference standards of CPC, DAC, DAOC and penicillin N analysed in the same conditions.

Analysis of the results showed that, after 7 days of fermentation, the transformant strains A. chrysogenum △cefEF T1, A. chrysogenum △cefEF T2 and A. chrysogenum △cefEF T3 accumulated a quantity of penicillin N equivalent to the sum of the quantities of CPC, DAC, DAOC and penicillin N produced by the parent strain. Therefore inactivation of the cefEF gene blocks the pathway for biosynthesis of cephalosporins without affecting the total quantity of β-lactams produced. The following table shows the percentages of each type of antibiotic out of the total of β-lactams produced by the different strains:

| Strain | CPC | DAC | DAOC | PenN |
|---|---|---|---|---|
| A. chrysogenum | 75.2 | 6.3 | 1.9 | 16.5 |
| A. chrysogenum △cefEF T1 | 0.0 | 0.5 | 0.0 | 99.5 |
| A. chrysogenum △cefEF T2 | 0.1 | 0.3 | 0.0 | 99.6 |
| A. chrysogenum △cefEF T3 | 0.1 | 0.2 | 0.0 | 99.7 |

### 2.3. Bioconversion of penicillin N to cephalosporins.

Enzymatic expansion of penicillin N was effected using resting cells or free extracts of cells of Streptomyces clavuligerus NP1. This strain hardly produces cephamycin at all when compared with the parent strain (Mahro and Demain, 1987), which facilitates determination of cephalosporins and/or cephamycins synthesized in vitro starting from penicillin N.

### 2.3.1. Enzymatic expansion using resting cells of S. clavuligerus NP1.

The inoculation medium MST (1% soluble starch, 3% trypticase soy broth without dextrose, 90 mM MOPS; pH 7.0) was sown with a suspension of spores of S. clavuligerus NP1 and was incubated at 200 rpm and 28°C for 48 hours. MST was also used as fermentation medium, inoculating in this case with 5% of the previous culture and incubating at 200 rpm and 28°C for 24 hours. Once the cultures had grown, the cells were harvested by centrifugation (10 000 rpm/ 10 min/ 4°C) and were washed twice with demineralized water. Finally, the cells were resuspended in 40 ml of demineralized water.

The reaction mixture (10 ml) for enzymatic expansion was prepared in accordance with described procedures (Shen et al. 1984.Enzyme Microbiol. Technol. 6: 402-404) using the penicillin N produced by A. chrysogenum △cefEF T1 as substrate: MOPS 50 mM pH 6.5, FeSO₄ (heptahydrate) 1.8 mM, α-ketoglutaric acid 2.56 mM, 8 ml of cellular suspension and penicillin N 0.2 mM. The reaction was incubated at 30°C and 200 rpm for 3 hours. 1 ml samples were taken at various times, and were centrifuged at 13 000 rpm for 10 minutes. The supernatants obtained were analysed by bioassay and HPLC.

The bioassay was carried out on plates of LB (2% agar) containing E. coli ESS as indicator microorganism and 50 000 UI/ml of penicillinase (Difco Bacto penase concentrate, Difco Laboratories, Detroit, MI). 200 µl of each sample was placed in wells prepared with the aid of a punch, and the plates were incubated at 37°C for 16 hours. The samples taken at time zero did not have halos of inhibition of the growth of E. coli ESS, whereas those incubated for 1 or 3 hours gave rise to inhibition halos of 17 and 21 mm respectively, indicating the presence of penicillinase-resistant reaction products (cephalosporins).

HPLC analysis was performed with Waters TAD equipment (Waters Associates, Milford, MA) with 486M1 detector, W600 pump, 717 autoinjector and a Nucleosil C₁₈-10 µm column (250x4.6 mm) (Phenomenex, Torrance, CA). The samples from the expansion reactions (50 µl) were analysed at a flow rate of 2.3 ml/min and a variable wavelength (214 or 254 nm). The analysis at 214 nm serves for determining the presence of penicillinases and at 254 nm for detecting cephalosporins. Elution was effected using, as the mobile phase, ammonium formate pH 3.3-methanol-tetrahydrofuran (99.85: 0.1: 0.05) in isocratic mode for 30 minutes. The analysis at 214 nm of the fermentation broth used as substrate revealed the existence of a peak at 10.1 minutes which coincides with the retention time obtained for penicillin N, whereas analysis at 254 nm did not reveal the existence of cephalosporins. Analysis of the reaction samples at time zero at 214 nm showed the presence of a peak at 10.1 minutes (penicillin N), whereas at 254 nm three peaks appeared with very short retention times (1.8-4 min) which did not coincide with those obtained for DAC (2.98 min), cephamycin C (3.3 min), DAOC (8.8 min) and CPC (17 min). The samples from 1-3 hours of reaction, analysed at 254 nm, showed a new peak relative to the chromatograms of the samples at time zero. This peak eluted at 8.8 minutes, coinciding with the retention time obtained for DAOC. This result demonstrates that the antibiotic produced by A. chrysogenum △cefEF T1 is penicillin N, which is expanded to DAOC by resting cells of S. clavuligerus.

### 2.3.2. Enzymatic expansion using free extracts of cells of S. clavuligerus NP1.

The MST inoculation medium (1% soluble starch, 3% trypticase soy broth without dextrose, 90 mM MOPS; pH 7.0) was sown with a suspension of spores of S. clavuligerus NP 1 and was incubated at 200 rpm and 28°C for 48 hours. The cells were collected by centrifugation (10 000 rpm/10 min/4°C), washed twice with buffer E (15 mM Tris HCl pH 7.5, 10% ethanol) and resuspended in buffer E containing DTT 10 mM and PMSF 2 mM. The cells were disrupted by sonication using a Labsonic 2000 (Braun Biotech) applying 3 pulses of 25 seconds. The sample was centrifuged at 20 000 rpm and 4°C for 20 minutes. The resulting extract was divided into aliquots which were frozen at -20°C and their protein content was analysed by the Bradford technique.

The reaction for enzymatic expansion was carried out by adding 100-200 µl of the protein extract to 0.5 ml of a reaction mixture that contained: Tris HCl 50 mM pH 7.5, ascorbic acid 4 mM, FeSO₄ (heptahydrate) 1.8 mM, α-ketoglutaric acid 1.28 mM and penicillin N produced by A. chrysogenum △cefEF T1 0.2 mM. The reaction was incubated at 30°C and 200 rpm for 2 hours and was stopped by addition of 1 volume of methanol and centrifugation at 13 000 rpm for 10 minutes. The supernatants obtained were analysed both by bioassay and by HPLC, as indicated in section 2.3.1.

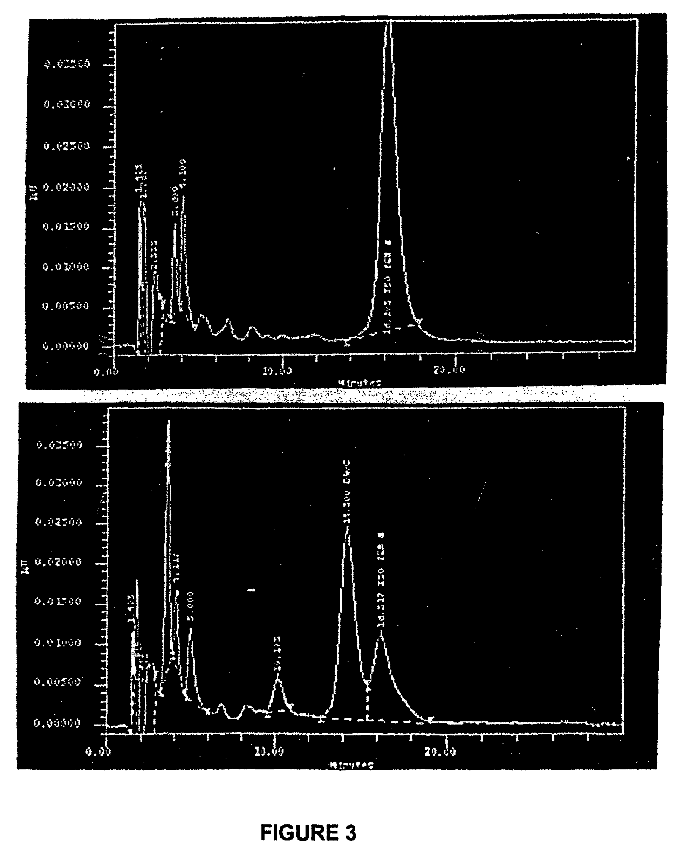

The samples taken at time zero did not produce halos of inhibition of the growth of E. coli ESS, whereas those incubated for 2 hours gave rise to inhibition halos, indicating the presence of penicillinase-resistant reaction products (cephalosporins). Moreover, analysis by HPLC at 214 nm of the reaction samples at time zero showed the presence of a peak at 10.1 minutes (penicillin N), whereas at 254 nm three peaks appeared with very short retention times (1.8-4 min) which did not coincide with those obtained for DAC (2.98 min), cephamycin C (3.3 min), DAOC (8.8 min) and CPC (17 min). The samples from 1-3 hours of reaction, analysed at 254 nm, showed a new peak relative to the chromatograms of the samples at time zero. This peak eluted at 8.8 minutes, coinciding with the retention time obtained for DAOC (Fig. 3). This result demonstrates that the antibiotic produced by A. chrysogenum △cefEF T1 is penicillin N, which is expanded to DAOC by an enzymatic extract of S. clavuligerus.

### 2.3.3. Enzymatic expansion using free extracts of cells of E. coli JM109 (DE3) pALE38.

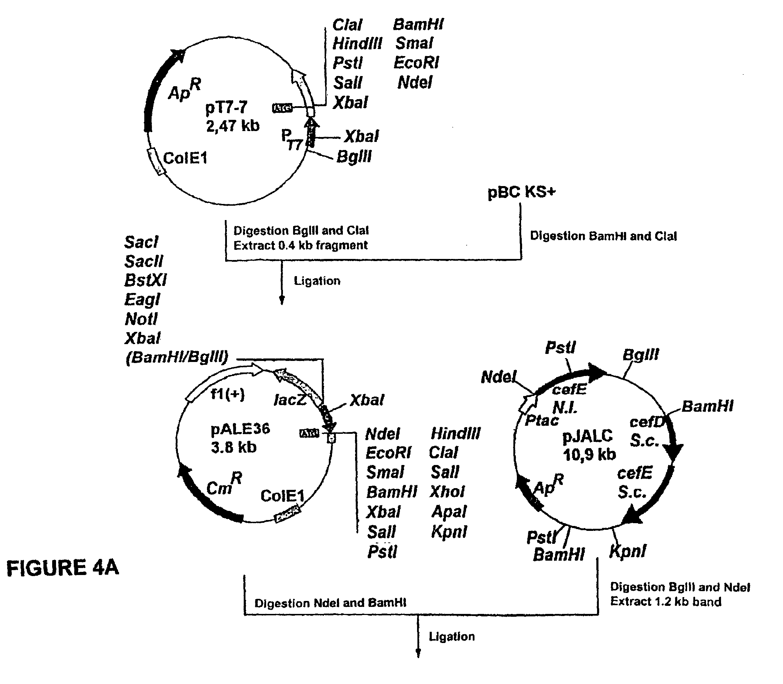

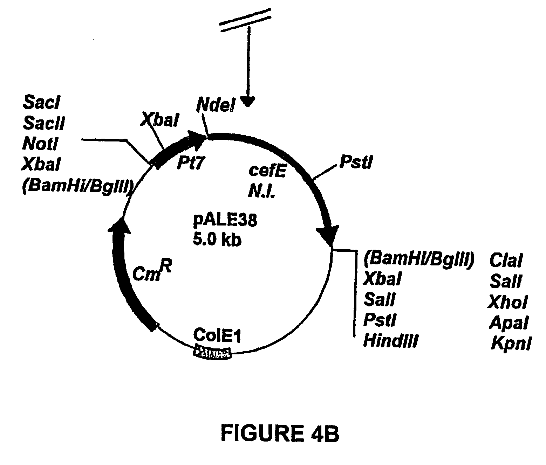

For expression of the cefE gene of Nocardia lactamdurans in E. coli, the plasmid pALE36 was constructed (Fig. 4A-B), which possesses a 400 pb fragment BglII-ClaI of the plasmid pT7-7 (Tabor and Richardson, 1985, Proc. Natl. Acad. Sci. USA 82: 1074-1078) in pBC KS+. This fragment contains a multiple cloning region situated downstream of the promoter of the Φ10 gene of the T7 bacteriophage. pALE36 has the advantage, relative to pT7-7, of using the Cm^{r} gene instead of the Ap^{r} gene. The latter codes for a β-lactamase activity that would degrade the penicillin N used as substrate. Next, pALE36 was digested with NdeI-BamHI and a 1.25 kb fragment NdeI-BglII that contained the cefE of N. lactamdurans was inserted. Finally, sequencing was used for verifying that the fusion between the promoter and the cefE gene was correct and with the plasmid obtained, pALE38, competent cells of E. coli/JM109(DE3) were transformed, selecting the transformant clones for resistance to chloramphenicol.

A colony of E. coli JM109 (DE3) pALE38 was used for inoculating a 250-ml flask with 50 ml of LB medium to which 30 µg/ml of chloramphenicol had been added (Sambrook, J. et al. 1989. Molecular cloning, a laboratory manual, Cold Spring Harbor Laboratory Press) and the culture was incubated at 250 rpm and 37°C for 14 hours. Next, 2 ml of this culture was used for sowing a 250 ml flask with 50 ml of LB-chloramphenicol medium and was incubated at 37°C and 250 rpm until O.D.₆₆₀ of 0.4 was reached. At this moment, IPTG was added to a final concentration of 1 mM and it was incubated for a further 6 hours. Processing of the cells obtained, the reaction of enzymatic expansion and analysis by bioassay and HPLC of the reaction products were carried out as indicated in section 2.3.2.

The samples taken at reaction time zero did not produce halos of inhibition of the growth of E. coli ESS, whereas those incubated for 2 hours gave rise to inhibition halos, indicating the presence of penicillinase-resistant reaction products. HPLC analysis at 214 nm of the reaction samples at time zero showed the presence of a peak at 10.1 minutes which corresponded to penicillin N. The samples incubated for 2 hours and analysed at 254 nm showed a new peak relative to the chromatograms of the samples at time zero. This peak eluted at 8.8 minutes, coinciding with the retention time obtained for DAOC. This result demonstrates that the antibiotic produced by A. chrysogenum △cefEF is penicillin N, which is expanded to DAOC by an enzymatic extract of E. coli JM109 (DE3) pALE38.

### 2.4. Determination of penicillin N by mass LC.

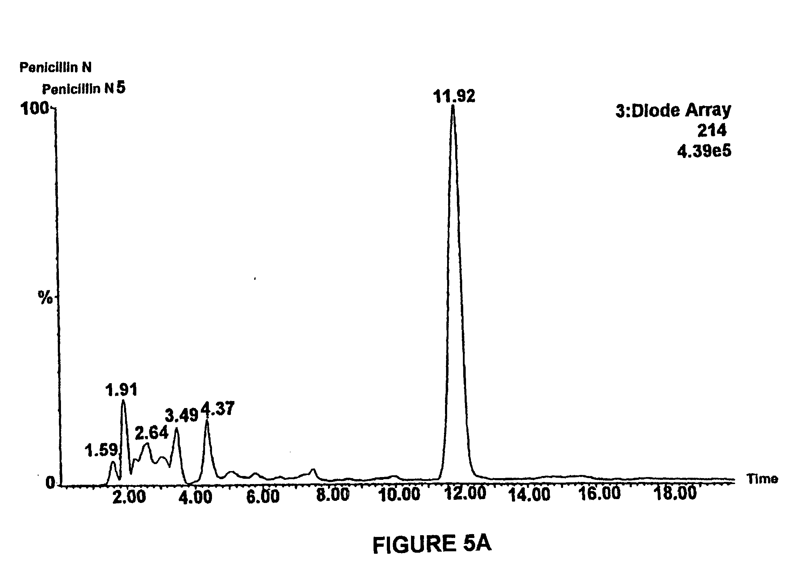

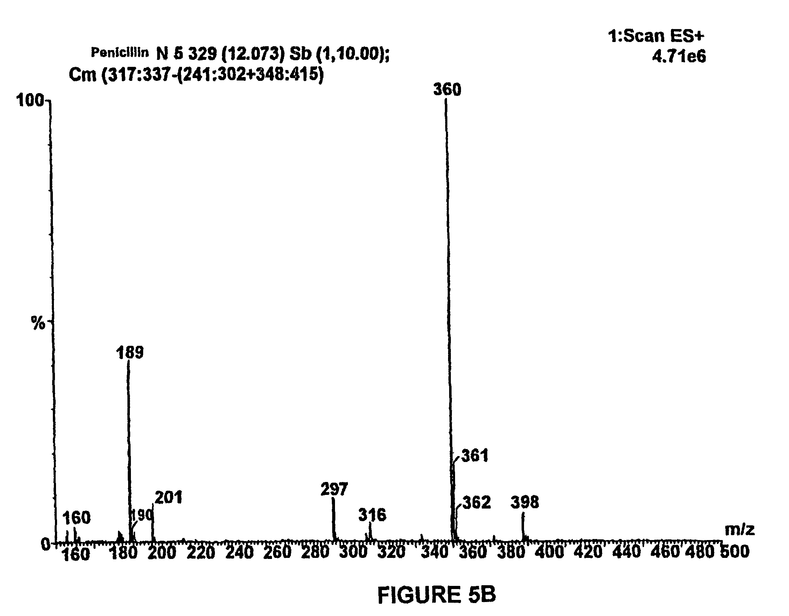

The samples from fermentation of the A. chrysogenum strain △cefEF were centrifuged for 5 minutes at 20 000 × g with the aim of removing the mycelium. Next, 50 µl of the supernatant was taken and was diluted 10 times with water, and 25 µl of this sample was analysed in a Waters 2690 TAD chromatograph (Waters Associates, Milford, MA) equipped with a Symmetry 300 C₁₈ 5 µm column (2.1 x 150 mm). The mobile phase used was ammonium formate buffer pH 3.3-methanol-tetrahydrofuran (99.85:0.1:0.05) with a flow rate of 0.25 ml/min. Analysis of the mass spectrum was performed with a Waters ZMD detector using the following parameters: detection range (996): 200-320 nm; capillary potential: 3.50 kV; cone potential: 40 V. Scan 1; 70 V. Scan 2; interphase: ES+. The spectrum obtained for the peak that elutes at 11.92 minutes coincides with that expected for penicillin N, exhibiting a molecular peak at 360 which corresponds to the molecular weight of penicillin N (Fig. 5A-B).

### EXAMPLE 3. Expression of the cefE gene in A. chrysogenum △cefEF.

With the aim of obtaining a strain of A. chrysogenum that produces DAOC by fermentation, the cefE gene, which codes for DAOCS activity, was expressed in A. chrysogenum △cefEF. The cefE genes cloned up to now originate from actinomycetes (S. clavuligerus, Nocardia lactamdurans and Lysobacter lactamgenus). For its expression in A. chrysogenum △cefEF, a series of plasmids was designed, in which the cefE genes of S. clavuligerus and N. lactamdurans were arranged under the control of promoters of fungal genes. Two promoters were chosen: one corresponding to a gene with high level of expression in fermentation conditions (pcbC gene, which codes for IPNS in P. chrysogenum) and the other belonging to the gpdA gene of A. nidulans, which is expressed constitutively and codes for glyceraldehyde 3-phosphate dehydrogenase activity.

The cluster formed by the fungal promoter, the cefE gene and the transcription terminator of the trpC gene of Aspergillus niger was inserted in the plasmid pALfleo7 (Barredo et al. 1997. Spanish Patent P9700482). This 5.4 kb plasmid possesses the phleomycin resistance gene of Streptoalloteichus hindustanus flanked by the promoter of the gdhA gene of P. chrysogenum and the transcription terminator of the trpC gene of Aspergillus niger.

### 3.1. Expression in A. chrysogenum △cefEF of the cefE gene of S. clavuligerus.

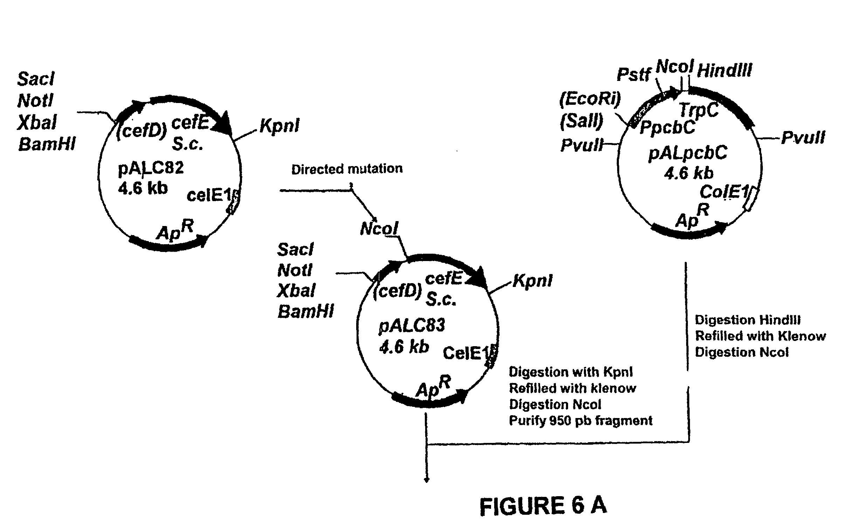

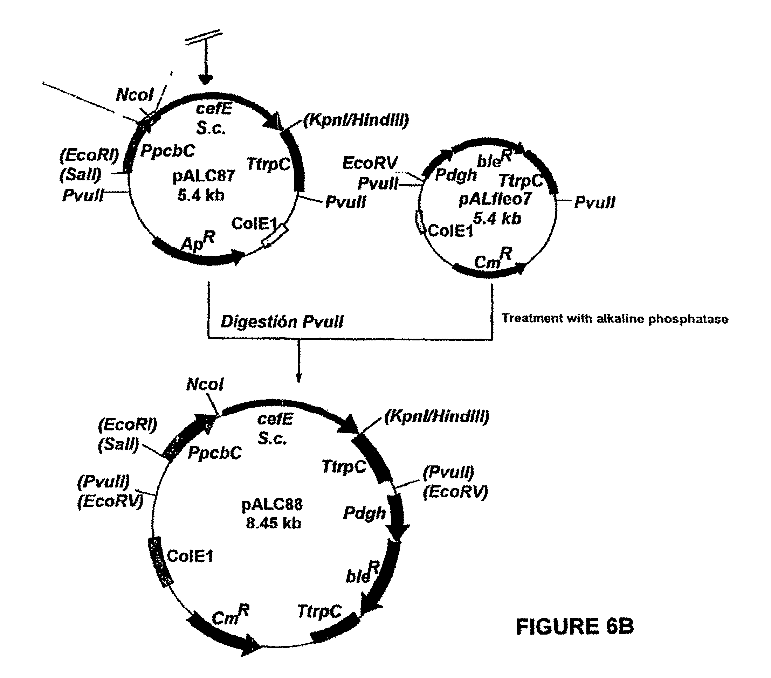

The cefE gene of S. clavuligerus was subcloned in the plasmid pBluescript I KS (+) (Stratagene) as a BamHI-KpnI fragment of 1.6 kb of the genome of S. clavuligerus that includes the complete cefE gene and 582 pb of the 3' end of the cefD gene. With the aim of expressing the cefE gene under the control of a fungal promoter, an NcoI restriction site was created at the 5' end of the said gene by means of directed mutation using the commercial kit QuikChange (Stratagene) and the oligonucleotides that are described in SEQ ID No. 1 and SEQ ID No. 2. The mutation process was carried out following the manufacturer's instructions and was verified by sequencing the creation of the NcoI restriction site and the absence of additional mutations. The plasmid resulting from this process was designated pALC83 (Fig. 6A-B). pALC83 was digested with KpnI, its ends were refilled with Klenow, next it was digested with Ncol and finally the 0.95 kb DNA fragment was purified using the QIAEX II kit (Qiagen). Then the 0.95 kb fragment was ligated with the plasmid pALpcbC (Fig. 6A-B) digested with HindIII, refilled with Klenow and digested with NcoI. The resulting plasmid was designated pALC87 and contains: (I) the cefE gene of S. clavuligerus expressed under the control of the promoter of the pcbC gene of Penicillium chrysogenum and (II) the terminator of the trpC gene of A. nidulans downstream of the cefE gene. Finally, pALC87 was digested with PvuII and the fragment that contained the cefE gene was ligated with pALfleo7 digested with EcoRV and dephosphorylated. As a result of this ligation, the plasmid pALC88 was obtained, which is the carrier of the insert PpcbC-cefE-TtrpC (Fig. 6A-B).

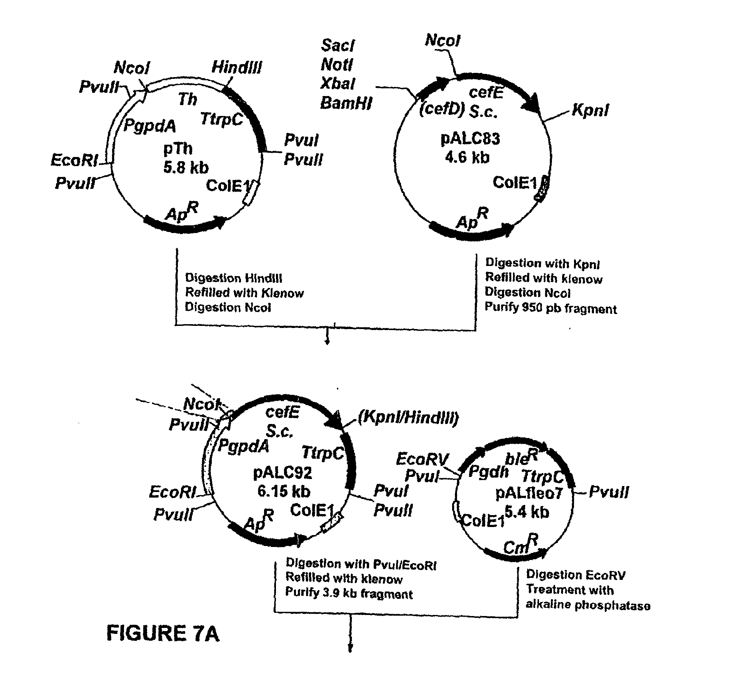

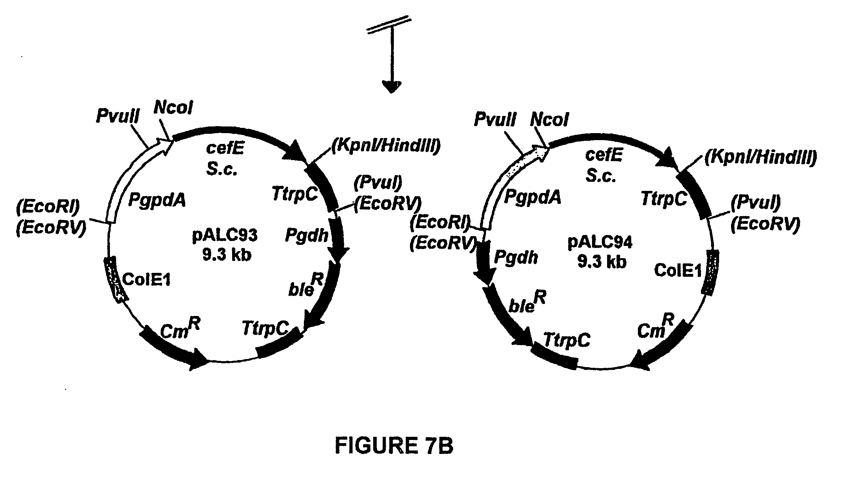

The cefE gene of S. clavuligerus was also expressed under the promoter of the gpdA gene of A. nidulans. For this, the 0.95 pb fragment that contains the cefE gene of S. clavuligerus used in the construction of pALC87 was ligated with the pTh plasmid (Uriach et al. 1995. EPA 0684312 A2) digested with HindIII, refilled with Klenow and then digested with NcoI. In the resulting plasmid (pALC92; Fig. 7A-B) the cefE gene of S. clavuligerus is localized under the control of the promoter of the gpdA gene of A. nidulans and followed by the terminator of the trpC gene of A. nidulans. The PgpdA-cefE-TtrpC expression cassette obtained by digestion of pALC92 with the enzymes EcoRI and PvuI and refilled with Klenow was inserted in pALfleo7 digested with EcoRV and dephosphorylated. As a result of this ligation, the plasmids pALC93 and pALC94 were obtained (Fig. 7A-B), in which the insert PpcbC-cefE-TtrpC occurs in the two possible orientations.

### 3.2. Expression in A. chrysogenum △cefEF of the cefE gene of N. lactamdurans.

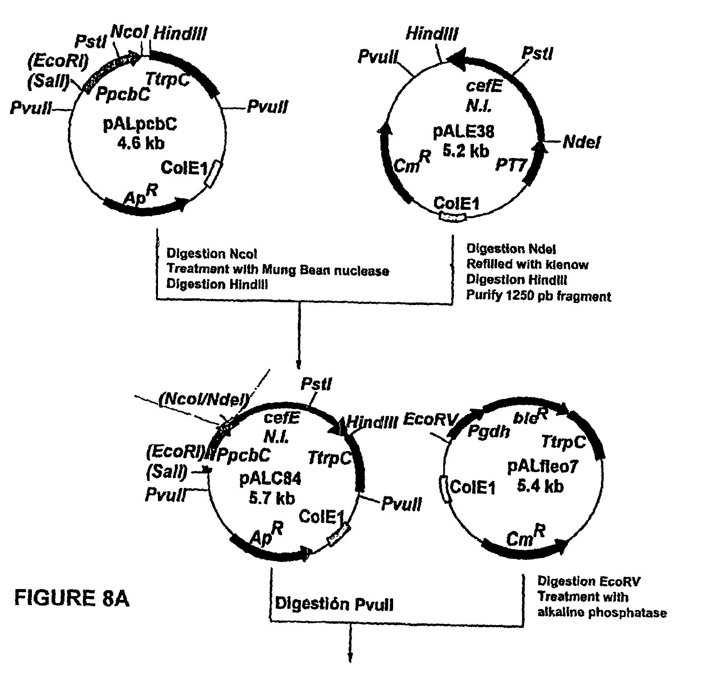

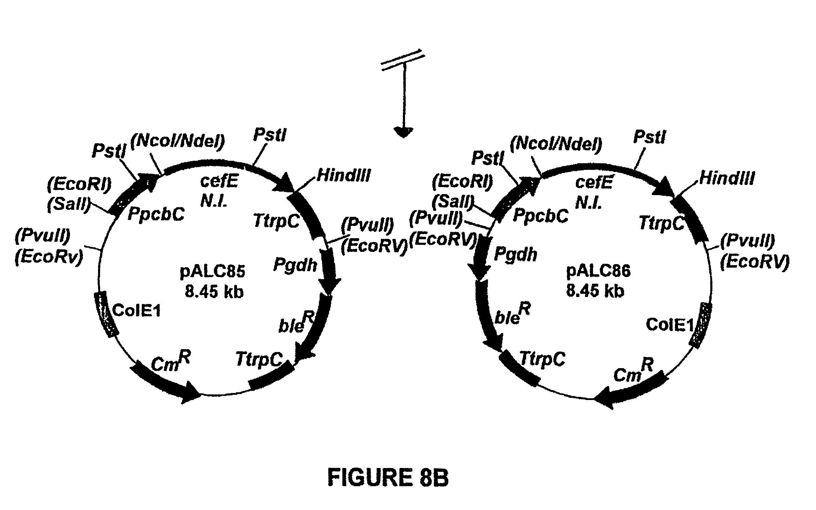

The cefE gene of N. lactamdurans was obtained by digestion of pALE38 (section 2.3.3) with NdeI, refilled with Klenow and digestion with HindIII. In this way a DNA fragment of 1250 pb was purified and was ligated with the plasmid pALpcbC (Fig. 6A-B) previously digested with NcoI, the projecting ends having been removed with Mung Bean nuclease and digested with HindIII. In the resulting plasmid (pALC84) the cefE gene of N. lactamdurans is under the control of the promoter of the pcbC gene of Penicillium chrysogenum and followed by the terminator of the trpC gene of A. nidulans. Fusion between the promoter and the cefE gene was confirmed by sequencing. pALC84 was then digested with the enzyme PvuII and the fragment generated was ligated with pALfleo7 previously digested with EcoRV and dephosphorylated. As a result of this ligation, the plasmids pALC85 and pALC86 were obtained (Fig. 8A-B), in which the insert PpcbC-cefE-TtrpC occurs in the two possible orientations. The strain that includes the pALC85 plasmid, designated E. coli DH5α /pALC85, was deposited in the Spanish Collection of Standard Cultures (CECT),University of Valencia, Research Building, Burjasot Campus, 46100 Burjasot (Valencia) with the No. 5150.

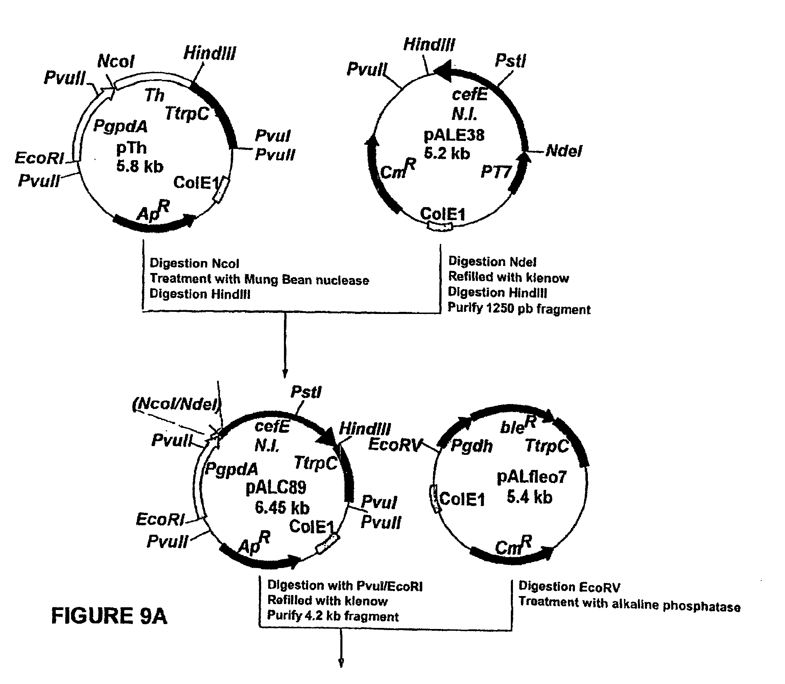

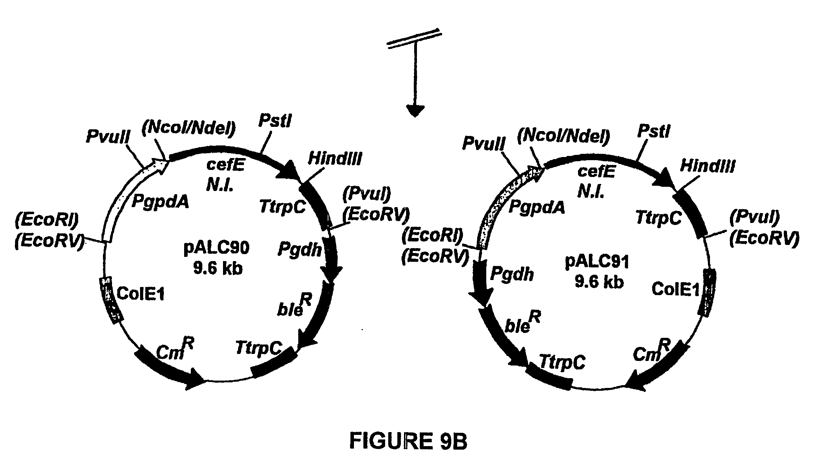

The cefE gene of N. lactamdurans was also expressed under the promoter of the gpdA gene of A. nidulans. For this, the DNA fragment of 1250 pb that contains the cefE gene of N. lactamdurans described previously was ligated with the plasmid pTh (Uriach et al. 1995. EPA 0684312 A2) digested with NcoI, the projecting ends being removed with Mung Bean nuclease and then digested with HindIII. In the resulting plasmid (pALC89; Fig. 9A-B) the cefE gene of N. lactamdurans is under the control of the promoter of the gpdA gene of A. nidulans and followed by the terminator of the trpC gene of A. nidulans. Fusion between the promoter and the cefE gene was confirmed by sequencing. The PgpdA-cefE-TtrpC expression cassette obtained by digestion of pALC89 with the enzymes EcoRI and PvuI and refilled with Klenow was inserted in pALfleo7 previously digested with EcoRV and dephosphorylated. As a result of this ligation, the plasmids pALC90 and pALC91 were obtained (Fig. 9A-B) in which the insert occurs in the two possible orientations.

### 3.3. Transformation in the strain A. chrysogenum △cefEF.

Three different strains of A. chrysogenum △cefEF (Tl, T2 and T3) were transformed with the plasmids pALC85, pALC86, pALC88, pALC90, pALC91, pALC93 and pALC94 in accordance with procedures previously described (Queener et al. 1985. Lieve (ed.) Microbiology-1985. pp. 468-472. ASM. Washington). The transformants were selected primarily for resistance to 3 µg/ml of phleomycin and then their resistance was tested at higher concentrations, on account of which descriptions exist that indicate that there is a direct correlation between level of resistance to phleomycin and number of copies of the plasmid integrated in the genome.

### 3.4. Selection of the transformants with greater production of DAOC.

The selected transformants were grown on plates of solid medium LPE (Le Page & Campbell, 1946, J. Biol. Chem. 162: 163-171) by incubating for 7 days at 28°C. Next, with the aid of a punch, a cylinder or plug was removed from each transformant, and this was bioassayed in a plate of LB medium to which had been added a culture of E. coli sensitive to β-lactams (E. coli ESS) and a preparation of penicillinase type I (Sigma) to a final concentration of 0.1 mg/ml. In this way, those transformants were selected that produced the largest inhibition halos, indicating that they were able to synthesize larger quantities of penicillinase-resistant antibiotics.

The results obtained are shown in the following table:

| Transformant No. | Strain/ plasmid | Phleomycin resistance (µg/ml) | Halo with penicillinase (mm) |
|---|---|---|---|
| 2800 | T1/pALC88 | 3 | 12 |
| 2801 | T2/pALC88 | 6 | 15 |
| 2802 | T3/pALC88 | 9 | 17 |
| 2803 | T1/pALC92 | 9 | 18 |
| 2804 | T2/pALC92 | 9 | 18 |
| 2805 | T3/pALC92 | 6 | 17 |
| 2806 | T1/pALC93 | 9 | 18 |
| 2807 | T2/pALC93 | 9 | 20 |
| 2808 | T3/pALC93 | 6 | 17 |
| 2809 | T1/pALC85 | 9 | 18 |
| 2810 | T2/pALC85 | 9 | 18 |
| 2811 | T3/pALC85 | 9 | 20 |
| 2812 | T1/pALC86 | 9 | 18 |
| 2810 | T2/pALC86 | 9 | 18 |
| 2811 | T3/pALC86 | 12 | 20 |
| 2812 | T1/pALC90 | 9 | 18 |
| 2813 | T2/pALC90 | 9 | 18 |
| 2814 | T3/pALC90 | 12 | 20 |
| 2815 | T1/pALC91 | 9 | 18 |
| 2816 | T2/pALC91 | 9 | 18 |
| 2817 | T3/pALC91 | 12 | 20 |

To analyse integration of the cefE gene in the genome of the transformants, total DNA was purified from the A. chrysogenum parent strain, from the strains A. chrysogenum △cefEF T1, A. chrysogenum △cefEF T2 and A. chrysogenum △cefEF T3 and from the transformants (A. chrysogenum △cefEF-cefE) selected by bioassay. The total DNA was digested with the enzymes BamHI and SalI and, after separating it by electrophoresis and transfer to nitrocellulose filters, it was hybridized using a 0.6 kb probe HindIII-KpnI corresponding to the cefE gene of N. lactamdurans (transformants 2800 to 2808) or with a 0.5 kb probe KpnI-ScaI corresponding to the cefE gene of S. clavuligerus (transformants 2809 to 2817). The result obtained showed the expected restriction template: the A. chrysogenum parent strain and the strains A. chrysogenum △cefEF T1, A. chrysogenum △cefEF T2 and A. chrysogenum △cefEF T3 did not show any hybridization band, whereas in the transformants designated A. chrysogenum △cefEF-cefE additional hybridization bands appeared, which indicated integration of the cefE gene in the genome of A. chrysogenum △cefEF.

### EXAMPLE 4. Production of DAOC by A. chrysogenum △cefE-cefE.

### 4.1. Fermentation of A. chrysogenum, A. chrysogenum △cefEF and A. chrysogenum △cefEF-cefE.

Fermentation of the strains of A. chrysogenum A. chrysogenum △cefEF and A. chrysogenum △cefEF-cefE was carried out as indicated in section 2.1, determining (I) that the antibiotic produced by A. chrysogenum △cefEF-cefE was DAOC, (II) the level of production of DAOC from the said transformant strains and (III) the possible presence of other antibiotics in the fermentation medium. The analyses that were carried out, as described below, were HPLC and mass LC.

### 4.2. Determination of the production of antibiotics by HPLC.

The fermentation samples were centrifuged for 5 minutes at 20 000 g to remove the mycelium and then 50 µl of the supernatant was diluted 10 times with water and analysed in a Waters TAD chromatograph (Waters Associates, Milford, MA) with 486M1 detector, W600 pump, 717 autoinjector and a Nucleosil C₁₈-10 µm column (250x4.6 mm) (Phenomenex, Torrance, CA). The samples were analysed at a flow rate of 2.3 ml/min and a wavelength of 254 nm. Elution was carried out using, as the mobile phase, ammonium formate pH 3.3-methanol-tetrahydrofuran (99.85: 0.1: 0.05) in isocratic mode for 30 minutes. Assignment of the peaks obtained to each compound was effected by comparison with reference standards of CPC, DAC, DAOC and penicillin N analysed in the same conditions.

Analysis of the results showed that, after 7 days of fermentation, the transformant strains A. chrysogenum △cefEF-cefE accumulated DAOC. The following table shows the percentages of each type of antibiotic out of the total of β-lactams produced by various strains:

| Strain | CPC | DAC | DAOC | PenN |
|---|---|---|---|---|
| A. chrysogenum | 75.2 | 6.3 | 1.9 | 16.5 |
| A. chrysogenum △cefEF T1 A. chrysogenum △cefEF-cefE | 0.0 | 0.5 | 0.0 | 99.5 |
| T3/pALC85 A. chrysogenum △cefEF-cefE | 0.1 | 0.3 | 95.9 | 3.7 |
| T3/pALC86 | 0.1 | 0.7 | 93.3 | 5.9 |
| A. chrysogenum △cefEF-cefE T3/pALC87 | 0.1 | 0.9 | 96.7 | 2.3 |

### 4.3. Purification of the DAOC produced by A. chrysogenum △cefEF-cefE.

A total of 10 litres of fermentation broth of A. chrysogenum △ cefEF-cefE containing 20 g/l of DAOC was adjusted to pH 3.0 with concentrated H₂SO₄ and was filtered through a Büchner filter with vacuum and with the aid of diatomaceous earth as filtration aid. The filtered biomass was washed with 10 litres of demineralized water in order to exhaust all the DAOC from the biomass, obtaining a volume of filtered broth of 16.4 litres. The filtered broth was passed through a column (1 m x 16 cm) of XAD4 polystyrene resin (Rohm & Haas) packed with 10 litres of resin at a flow rate of 150 ml/min. The column was washed with approximately 10 litres of water and was eluted with a 0.15 M sodium acetate solution. The eluted fractions were analysed by HPLC to determine the DAOC content. Then the DAOC-rich fractions were combined, obtaining a volume of eluate of 23.5 litres.

The eluate was adjusted to pH 5.5 and was submitted to decolorizing and purification by percolation through a column packed with anion-exchange resin IRA68 (Rohm & Haas). The decolorized solution was submitted to a concentration process by reverse osmosis until a DAOC concentration above 200 g/l was reached. Next, 1.1 litres of the concentrate from reverse osmosis was precipitated with stirring by slowly adding 4 litres of isopropyl alcohol. After 2 hours of precipitation, it was filtered through a porous glass plate and the precipitate obtained was washed with approximately 1 litre of isopropyl alcohol. The precipitate was dried in a vacuum stove at 50°C, obtaining 147.3 g of DAOC sodium salt with a purity of 92%.

### EXAMPLE 5. Bioconversion of DAOC to glutaryl-7-ADCA.

### 5.1. Bioconversion of DAOC to glutaryl-7-ADCA catalysed by immobilized DAO.

To carry out the bioconversion of the DAOC obtained as described in Example 4, 66 g of DAOC sodium salt was dissolved in 2 litres of 25 mM potassium phosphate buffer containing 0.5 g of sodium metabisulphite. The solution of DAOC was placed in a 3-litre reactor containing 150 g of DAO immobilized on Eupergit C (Röhm) according to methods described (Cambiaghi et al. 1991. EP 496993). The mixture was incubated at 25°C with light stirring and bubbled with oxygen (1 vol./vol./min) through a diffuser located at the bottom of the reactor. The pH was maintained at 7.5 by automatic addition of ammonium hydroxide at 5%. In 75 minutes the DAOC had been converted completely, yielding a mixture of glutaryl-7-ADCA (90%) and ketoadipyl-7-ADCA (10%).

To convert the residual ketoadipyl-7-ADCA to glutaryl-7-ADCA, the solution obtained after incubation was separated by filtration of the immobilized enzyme and 10 ml of hydrogen peroxide (H₂O₂) was added to a concentration of 3.5% per litre of filtrate. The mixture was incubated for 15 minutes at 25°C, after which 0.5 g of sodium pyruvate was added. After this treatment the ketoadipyl-7-ADCA is completely converted to glutaryl-7-ADCA, obtaining a solution of glutaryl-7-ADCA at 3%. The said solution was concentrated by osmosis at 4°C to a concentration of 5%, for use in the next enzymatic stage.

### 5.2. Bioconversion of DAOC to glutaryl-7-ADCA catalysed by intact cells of Rhodotorula gracilis.

A total of 100 litres of fermentation broth of A. chrysogenum △cefEF-cefE containing 35 g/l of DAOC was adjusted to pH 7.5 and, in the same fermenter, 5 kg of concentrate of cells of Rhodotorula gracilis ATCC 26217 was added, corresponding to 500 000 units of DAO according to methods previously described (Cambiaghi et al. 1991. EP 496993). The reaction of bioconversion proceeded at 25°C with light stirring while adding oxygen at 1 vol./vol./min through the diffuser located at the bottom of the fermenter. The pH was maintained at pH 7.5 by automatic addition of ammonia at 5% and the conversion of DAOC to glutaryl-7-ADCA was completed at the end of 90-120 minutes.

Next the fermentation solids were separated, containing mainly cells of A. chrysogenum and of Rhodotorula gracilis together with variable amounts of insoluble material of the fermentation medium, by using a system of ultrafiltration through membranes with a molecular cutoff of 30 000 Da. On completion of ultrafiltration, and with the aim of converting the residual ketoadipyl-7-ADCA to glutaryl-7-ADCA, 5 ml of hydrogen peroxide (H₂O₂) was added with stirring, to a concentration of 3.5% per litre of filtrate. The mixture was incubated for 15 minutes at 25°C, after which 50 g of sodium pyruvate was added. After this treatment, all the ketoadipyl-7-ADCA had been converted to glutaryl-7-ADCA, obtaining a solution with a level of glutaryl-7-ADCA of 1%. The said solution was concentrated by reverse osmosis to an approximate concentration of 5% of glutaryl-7-ADCA.

### 5.3. Extraction of glutaryl-7-ADCA

1 litre of isobutyl acetate was added to 1 litre of the solution of glutaryl-7-ADCA, stirring vigorously, and cooling at a temperature between 0 and 5°C. Next, 2 N H₂SO₄ was added while stirring until the solution reached a pH of 1.5 and the phases separated rapidly. This operation was repeated with 1 litre of fresh isobutyl acetate, maintaining the temperature between 0 and 5°C to exhaust the aqueous phase of glutaryl-7-ADCA. Both extracts of rich isobutyl acetate were combined and they were extracted with 0.75 litres of 25 mM phosphate buffer at pH 8.0. The solution was adjusted to pH 8.0 by adding 2 N KOH and the phases separated. The organic phase was washed with 0.25 litres of 25 mM phosphate buffer at pH 8.0 with the aim of finally exhausting it of glutaryl-7-ADCA. The aqueous extracts obtained, containing 50 g/l of glutaryl-7-ADCA, were combined and were used directly for the subsequent enzymatic conversion or they were submitted to a process of vacuum distillation to remove the residues of organic solvent and thus increase the life of the catalyst in the subsequent enzymatic conversion.

### EXAMPLE 6. Bioconversion of glutaryl-7-ADCA to 7-ADCA.

### 6.1. Conversion of glutaryl-7-ADCA to 7-ADCA catalysed by immobilized glutaryl acylase.

A total of 150 ml of solution of glutaryl-7-ADCA containing 7.5 g of glutaryl-7-ADCA was incubated at 25°C in the presence of 30 g of glutaryl acylase immobilized on Eupergit C (31 U/g wet). The reaction mixture was incubated with stirring for 60 minutes, maintaining the pH at 8.0 by automatic addition of ammonia solution at 5%. At the end of the said time, conversion of glutaryl-7-ADCA to 7-ADCA was found to exceed 98%. The biocatalyst was separated from the solution by filtration, and was then washed with 25 ml of water, and was reused for a minimum of 100 cycles for new bioconversions (Cambiaghi et al. 1991. EP 496993).

### 6.2. Recovery of 7-ADCA.

A total of 175 ml of solution from conversion of glutaryl-7-ADCA to 7-ADCA was treated with 3 g of active carbon for 20 minutes at room temperature and with stirring with the aim of removing impurities and reducing the colour of the end product. The carbon was removed by filtration and washed with 10 ml of water. The filtered solution was adjusted to pH 4.5 by adding 6 N H₂SO₄, so that crystallization of 7-ADCA was initiated, and it was incubated for 15 minutes with slow stirring so that crystallization proceeded. The temperature was lowered to 5-10°C, finally adjusting the pH to 3.8 by addition of 6 N H₂SO₄. After 2 hours of incubation, the precipitate was recovered by filtration and was washed with 25 ml of water. The yield in crystallization was 95.5%, obtaining a 7-ADCA with a purity of 98.3%.

### 6.3. Purification and crystallization of 7-ADCA.

125 ml of dichloromethane was added to a total of 875 ml of solution of 7-ADCA, the temperature was lowered to 0-5°C and the pH was adjusted to 0.8 quickly with stirring by adding 6 N H₂SO₄. On reaching pH of 0.8, the phases were separated by decanting or centrifugation, the aqueous phase being washed with a further 125 ml of dichloromethane. This operation has the purpose of removing substances that are hydrophobic and of an acidic character, which are extracted by dichloromethane in these conditions, making it possible to achieve greater purity of the end product. The aqueous phase thus obtained was treated with 3 g of active carbon for 20 minutes with stirring to remove impurities and reduce the colour of the end product. The carbon was removed by filtration and washed with 10 ml of water. The filtered solution was adjusted to pH 3.0 by addition of 2 N ammonia solution so that crystallization of the 7-ADCA was initiated. Next, it was incubated for 15 minutes with slow stirring until crystallization progressed, finally adjusting the pH to 3.7 by addition of 2 N NH₄OH and to a temperature between 5-10°C. After 2 hours the precipitate was recovered by filtration and was washed with 25 ml of water. The yield in crystallization was 94%, obtaining a 7-ADCA with a purity of 98.5%.

### EXAMPLE 7. Production of 7-ADCA by A. chrysogenum △cefEF-cefE-dao-gla and A. chrysogenum △cefEF-cefE-cac.

Taking a previous description as a basis, it is possible to construct a biosynthetic operon for the production of 7-ACA directly by fermentation of A. chrysogenum (Isogai et al. 1991. Biotechnology 9: 188-91). The system described is based on the expression in A. chrysogenum of the genes (cDNAs) that code for the enzymatic activities DAO and GLA of Fusarium solani and Pseudomonas diminuta respectively. Applying the same idea to the strain A. chrysogenum △cefEF-cefE, it is possible to construct a biosynthetic operon for the production of 7-ADCA directly by fermentation of A. chrysogenum △cefEF-cefE-dao-gla. In this case, as it is impossible for A. chrysogenum △cefEF-cefE to produce CPC, the biosynthesis of 7-ADCA takes place instead of 7-ACA. The 7-ADCA produced by fermentation of A. chrysogenum △cefEF-cefE-dao-gla can be purified as indicated in Example 6.

### DESCRIPTION OF THE DIAGRAMS

Fig. 1A-B. Scheme for construction of pALC73, the plasmid for disruption of the cefEF gene of A. chrysogenum. Firstly, the plasmid pExp7.2 BB, which includes the cefEF and cefG genes of A. chrysogenum, was subcloned at the BamHI site of the plasmid pBC KS (+) at which the XhoI site had been inactivated previously. The resulting plasmid was designated pALC72. Next the hygromycin-resistance gene (hygR) was inserted in the XhoI site of the cefEF gene giving rise to pALC73. This 14.6 kb plasmid includes the chloramphenicol-resistance gene (CmR) as selection marker in E. coli and the hygromycin-resistance gene (hygR) as selective marker in A. chrysogenum. The hygR gene is expressed under the control of the promoter gpd (glyceraldehyde 3 phosphate dehydrogenase) and possesses the terminator trpC (tryptophan C).

Fig. 2. Scheme of the disruption of the cefEF gene of A. chrysogenum. The plasmid pALC73 is integrated by double recombination in one of the chromosomes of A. chrysogenum inactivating the cefEF gene. The lower part of the diagram shows Southern analysis of the clones of A. chrysogenum disrupted at the cefEF gene. The hybridization band obtained by BamHI digestion of the DNA of the parent strain is converted to two bands of 5.7 kb and 5.3 kb in the transformant strains with the inactivated cefEF gene. Similarly, the 0.5 kb hybridization band SalI of the parent strain is converted to another 2.2 kb band in the transformants disrupted in the cefEF gene. The disrupted transformants are designated A. chrysogenum △cefEF T1, A. chrysogenum △cefEF T2 and A. chrysogenum △cefEF T3. P corresponds to the A. chrysogenum parent strain, whereas T1, T2 and T3 are transformants of A. chrysogenum with the cefEF gene inactivated.

Fig. 3. Enzymatic expansion of penicillin N to DAOC using free enzymatic extracts of cells of S. clavuligerus NP1. The top part shows a chromatogram of the reaction mixture at time 0 and the bottom part shows the same mixture after 2 hours of reaction. Abscissa: time in minutes; Ordinate: Optical Density (O.D.)

Fig. 4A-B. Scheme of construction of pALE38, the plasmid for expression of the cefE gene of Nocardia lactamdurans in E. coli under the control of the promoter T7.

Fig. 5A-B. Mass LC analysis of penicillin N produced by A. chrysogenum △cefEF T1. The top part shows a chromatogram at 214 nm and the bottom part shows the mass spectrum corresponding to the peak that elutes at 11.92 minutes.

Fig. 6A-B. Scheme of construction of pALC88, the plasmid for expression of the cefE gene of S. clavuligerus in A. chrysogenum under the control of the promoter of the pcbC gene of P. chrysogenum.

Fig. 7A-B. Scheme of construction of pALC93 and pALC94, plasmids for expression of the cefE gene of S. clavuligerus in A. chrysogenum under the control of the promoter of the gpdA gene of A. nidulans.

Fig. 8A-B. Scheme of construction of pALC85 and pALC86, plasmids for expression of the cefE gene of N. lactamdurans in A. chrysogenum under the control of the promoter of the pcbC gene of P. chrysogenum.

Fig. 9A-B. Scheme of construction of pALC90 and pALC91, plasmids for expression of the cefE gene of N. lactamdurans in A. chrysogenum under the control of the promoter of the gpdA gene of A. nidulans.

## Claims

1. A process for producing 7-aminodesacetoxycephalosporanic acid (7-ADCA), **characterized in that** it comprises the following operations:
a) Inactivating, in a cephalosporin C-producing strain of A. chrysogenum, the enzymatic desacetoxycephalosporin C synthase /desacetylcephalo-sporin C synthase (DAOCS/DACS) activity encoded by the cefEF gene, by disruption of the said gene, constructing a penicillin N-producing strain.
b) Expressing, in the said penicillin N-producing strain, the cefE gene that codes for the enzymatic activity desacetoxycephalosporin C synthase (DAOCS), constructing a strain that is a producer of desacetoxycephalosporin C (DAOC).
c) Growing the DAOC-producing strain in conditions of production of DAOC and purifying the DAOC produced.
d) Bioconverting the DAOC produced to glutaryl-7-ADCA by using the enzyme D-amino-acid oxidase (DAO).
e) Bioconverting the glutaryl-7-ADCA to 7-ADCA by using the enzyme glutaryl acylase (GLA).
f) Purifying the 7-ADCA produced in the bioconversion.

2. A method according to Claim 1, **characterized in that** stages d) and e) are carried out by means of the expression, in a strain of A. chrysogenum in which the cefEF gene has previously been inactivated and the cefE gene expressed, of the dao and gla genes, which code for the enzymatic activities D-amino-acid oxidase (DAO) and glutaryl acylase (GLA), respectively, giving rise to a 7-ADCA-producing strain and growing the said strain in conditions of production of 7-ADCA.

3. A method according to Claim 1, **characterized in that** stages d) and e) are carried out by means of the expression, in a strain of A. chrysogenum in which the cefEF gene has previously been inactivated and the cefE gene expressed, of the cac gene, which codes for the enzymatic cephalosporin acylase (CAC) activity, giving rise to a 7-ADCA-producing strain and growing the said strain in conditions of production of 7-ADCA.

4. A process for producing penicillin N, **characterized in that** any of the operations described in Claims 1 to 3 are interrupted once stage a) has ended.

5. A DNA compound, defined by the restriction map of pALC73, that includes the cefEF gene inactivated by the hygromycin-resistance gene (hygR).

6. Vectors that carry the DNA compounds described in Claim 5 or fragments thereof.

7. Vectors according to Claim 6, **characterized in that** they consist of a plasmid.

8. Plasmid according to Claim 7, **characterized in that** it consists of pALC73.

9. Transformed host microorganisms, **characterized in that** they possess the cefEF gene that has been disrupted by introduction of the DNA compounds of Claim 5, included completely or partially in the vectors of Claims 6 to 8.

10. Transformed host microorganism according to Claim 9, **characterized in that** it consists of a prokaryote, preferably E. coli or an actinomycete.

11. Transformed host microorganism according to Claim 10, **characterized in that** it consists of pure strains of E. coli represented by CECT5151 or its mutants or transformed derivatives.

12. Transformed host microorganism according to Claim 9, **characterized in that** it consists of a eukaryote, preferably of the genus Acremonium or Penicillium.

13. Transformed host microorganism according to Claim 12, **characterized in that** it preferably consists of Acremonium chrysogenum or Penicillium chrysogenum.

14. Transformed host microorganism according to Claim 13, **characterized in that** it consists of pure strains of Acremonium chrysogenum or its mutants and transformed derivatives.

15. A method of obtaining transformed microorganisms with improved production of penicillin N, **characterized in that** it comprises the following operations:
a) Constructing vectors that carry, completely or partially, the DNA compound described in Claim 5 or recombinant DNA compounds thereof.
b) Introducing the aforesaid vectors in A. chrysogenum, inactivating the function of the cefEF gene.
c) Selecting the transformed microorganisms that exhibit increased production of penicillin N when compared with control microorganisms without transformation.

16. A method of obtaining transformed microorganisms according to Claim 15, **characterized in that** the vectors constructed are those defined in Claims 6 to 8.

17. A method according to Claim 16 in which the transformed host microorganisms are those defined in Claims 9 to 14.

18. A method of production of desacetoxycephalosporin C (DAOC), **characterized in that** any of the processes described in Claims 1 to 3 are interrupted once stage c) has ended.

19. A method according to Claim 18, in which a DNA compound defined by the restriction map of pALC88, which includes the cefE gene of S. clavuligerus expressed under the control of the promoter of the pcbC gene of P. chrysogenum, is used in stage b).

20. A method according to Claim 18, in which a DNA compound defined by the restriction map of pALC85, which includes the cefE gene of N. lactamdurans expressed under the control of the promoter of the pcbC gene of P. chrysogenum, is used in stage b).

21. A method according to Claim 18, in which a DNA compound defined by the restriction map of pALC86, which includes the cefE gene of N. lactamdurans expressed under the control of the promoter of the pcbC gene of P. chrysogenum, is used in stage b).

22. A method according to Claim 18, in which a DNA compound defined by the restriction map of pALC93, which includes the cefE gene of S. clavuligerus expressed under the control of the promoter of the gpdA gene of A. nidulans, is used in stage b).

23. A method according to Claim 18, in which a DNA compound defined by the restriction map of pALC94, which includes the cefE gene of S. clavuligerus expressed under the control of the promoter of the gpdA gene of A. nidulans, is used in stage b).

24. A method according to Claim 18, in which a DNA compound defined by the restriction map of pALC90, which includes the cefE gene of N. lactamdurans expressed under the control of the promoter of the gpdA gene of A. nidulans, is used in stage b).

25. A method according to Claim 18, in which a DNA compound defined by the restriction map of pALC91, which includes the cefE gene of N. lactamdurans expressed under the control of the promoter of the gpdA gene of A. nidulans, is used in stage b).

26. Vectors that carry the DNA compounds described in Claims 19 to 25 or fragments thereof.

27. Vectors according to Claim 26, **characterized in that** they consist of a plasmid.

28. Plasmids according to Claim 27, **characterized in that** they consist of pALC85, pALC86, pALC88, pALC90, pALC91, pALC93 or pALC94.

29. Transformed host microorganisms, **characterized in that** they possess the disrupted cefEF gene and **in that** they produce penicillin N, in which the cefE gene has been expressed by introducing the DNA compounds of Claims 19 to 25, included completely or partially in the vectors of Claims 26 to 28.

30. Transformed host microorganism according to Claim 29, **characterized in that** it consists of a prokaryote, preferably E. coli or an actinomycete.

31. Transformed host microorganism according to Claim 30, **characterized in that** it consists of pure strains of E. coli represented by CECT5150 or its mutants or transformed derivatives.

32. Transformed host microorganism according to Claim 29, **characterized in that** it consists of a eukaryote, preferably of the genus Acremonium or Penicillium.

33. Transformed host microorganism according to Claim 32, **characterized in that** it consists preferably of Acremonium chrysogenum or Penicillium chrysogenum.

34. Transformed host microorganism according to Claim 33, **characterized in that** it consists of pure strains of Acremonium chrysogenum or its mutants and transformed derivatives.

35. A method of obtaining transformed microorganisms with improved production of desacetoxycephalosporin C (DAOC), **characterized in that** it comprises the following operations:
a) Constructing vectors that carry, completely or partially, the DNA compounds described in Claims 19 to 25 or recombinant DNA compounds thereof.
b) Introducing the said vectors in a strain of A. chrysogenum whose cefEF gene has been inactivated.
c) Selecting the transformed microorganisms that exhibit increased production of desacetoxycephalosporin C (DAOC) when compared with control organisms without transformation.

36. A method of obtaining transformed microorganisms according to Claim 35, **characterized in that** the vectors constructed are those defined in Claims 26 to 28.

37. A method according to Claim 36, in which the transformed microorganisms are those defined in Claims 29 to 34.
